# EUROPEAN PATENT APPLICATION

(11) **EP 1 466 978 A1**
(43) Date of publication of application: **13.10.2004**
(21) Application number: 02791977.8
(22) Date of filing: 20.12.2002
(51) Int. Cl.: C12N 15/29, C12N 15/09, C12N 5/14, A01H 5/00

(54) **CHITIN OLIGOSACCHARIDE ELICITOR AND GIBBERELLIN RESPONSIVE GENES IN PLANT AND UTILIZATION THEREOF**

(30) Priority: 20.12.2001 JP 2001387862
(71) Applicant: NATIONAL INSTITUTE OF AGROBIOLOGICAL SCIENCES, Tsukuba-shi, Ibaraki-ken 305-8602 (JP)
(72) Inventor: MINAMI, Eiichi, Tsukuba-shi, Ibaraki 305-0044 (JP); Shibuya, N., Nat. Inst. of Agrobiological Sciences, Tsukuba-shi, Ibaraki 305-8602 (JP); Day, R.B., Nat. Inst. of Agrobiological Sciences, Tsukuba-shi, Ibaraki 305-8602 (JP)
(74) Representative: Winckels, Johannes Hubertus F.
(86) International application number: PCT/JP2002/013375
(87) International publication number: WO 2003/054196

(57) **Abstract**

Genes whose expression was induced in the early stages of elicitor treatment were investigated using a DNA microchip containing 1265 varieties of rice ESTs. A chitin oligomer (N-acetylchitooligosaccharide), an important component in the cell wall of rice blast fungus, was used as the elicitor. This resulted in the identification of six varieties of novel elicitor-responsive ESTs. Of these, the product of two genes (named CIGR1 gene and CIGR2 gene) possessed a motif characteristic of the GRAS family, which are thought to be transcription factors. Thus it was indicated for the first time that, in addition to gibberellin signal transduction regulatory factors, the GRAS family is also present in rice.

SEQUENCE LISTING

<110> National Institute of Agrobiological Sciences

<120> CHITIN OLIGOSACCHARIDE ELICITOR- AND GIBBERELLIN-RESPONSIVE GENES IN
PLANTS, AND USES THEREOF

<130> MOA-A0107P

<140>
<141>

<150> JP 2001-387862
<151> 2001-12-20

<160> 6

<170> PatentIn Ver. 2.1

<210> 1
<211> 2135
<212> DNA
<213> Oryza sativa L cv Nipponbare

<220>
<221> CDS

<222> (137)..(1852)

<400> 1

gcacgagaga gaaccagaga gagagagaga gagagagaga gagagagagg agtggaagtg 60

gatcggggca tcgctgcggc gtcggggctg tgcagatcgg aggaggtcat gaacgactaa 120

tctccgttca tttatt atg gac ttg cac cag tta tta aag tac aga ttg act 172

Met Asp Leu His Gln Leu Leu Lys Tyr Arg Leu Thr

1               5                   10

ggc gct aac gtt gtg tac gaa att cct aca gag aac aac ttg caa aac 220

Gly Ala Asn Val Val Tyr Glu Ile Pro Thr Glu Asn Asn Leu Gln Asn

        15                  20                  25

tct ccc tgg caa gct aac cca ctg aag tac gag ttc agc gat tcc cca 268

Ser Pro Trp Gln Ala Asn Pro Leu Lys Tyr Glu Phe Ser Asp Ser Pro

    30                  35                  40

tac acc cct ctc tct tcc caa ttt gag tgt gac aat ttg tct gct ctt 316

Tyr Thr Pro Leu Ser Ser Gln Phe Glu Cys Asp Asn Leu Ser Ala Leu

45                  50                  55                  60

acc aac act cca gat aac cag agc tct aca gaa acc att tca gcc caa 364

Thr Asn Thr Pro Asp Asn Gln Ser Ser Thr Glu Thr Ile Ser Ala Gln

                65                  70                  75

cca atc tcc cca ttg gaa gca gac agc tca tat aga cag gcg ggt att 412
Pro Ile Ser Pro Leu Glu Ala Asp Ser Ser Tyr Arg Gln Ala Gly Ile
            80                  85                  90

ctt ctc cag gag aac att caa gtg gga gct gat ccg ttg tat gct aca 460
Leu Leu Gln Glu Asn Ile Gln Val Gly Ala Asp Pro Leu Tyr Ala Thr
        95                  100                 105

tca aga cat aat atg caa cac gcc tta cgg gag att gag act gtt ctg 508
Ser Arg His Asn Met Gln His Ala Leu Arg Glu Ile Glu Thr Val Leu
    110                 115                 120

atg gca cct gat aca gat gat gca aca act agc acc aag cat gag ttt 556
Met Ala Pro Asp Thr Asp Asp Ala Thr Thr Ser Thr Lys His Glu Phe
125                 130                 135                 140

gag gaa atc aag cct gct cag ctt gtg agg cag cgg tcg aga aca tgg 604
Glu Glu Ile Lys Pro Ala Gln Leu Val Arg Gln Arg Ser Arg Thr Trp
                145                 150                 155

agt cat gaa tca cgg cag ccg tta cct gga gtt ggt cgg tca cag ttt 652
Ser His Glu Ser Arg Gln Pro Leu Pro Gly Val Gly Arg Ser Gln Phe
            160                 165                 170

gca tct ggt gga tac ccc aca gca agc tat gaa ttt cgt cca gag aaa 700

Ala Ser Gly Gly Tyr Pro Thr Ala Ser Tyr Glu Phe Arg Pro Glu Lys
        175                 180                 185

cgg caa agg gag tta agg gaa gac cct cag ata att gtg aag cag cta 748
Arg Gln Arg Glu Leu Arg Glu Asp Pro Gln Ile Ile Val Lys Gln Leu
    190                 195                 200

tta acc agg tgt gct gag gct ctg agt gag gac agg aca gag gag ttc 796
Leu Thr Arg Cys Ala Glu Ala Leu Ser Glu Asp Arg Thr Glu Glu Phe
205                 210                 215                 220

cat aag ctt gtt cag gag gct cgt gga gtg gtc tca atc aac ggg gaa 844
His Lys Leu Val Gln Glu Ala Arg Gly Val Val Ser Ile Asn Gly Glu
                225                 230                 235

cca atc caa cgt cta ggt gct tac cta ctg gag ggt ttg gtt gct aga 892
Pro Ile Gln Arg Leu Gly Ala Tyr Leu Leu Glu Gly Leu Val Ala Arg
            240                 245                 250

cat gga aac tct ggc aca aac atc tac cgt gct ctg aag tgc cgt gag 940
His Gly Asn Ser Gly Thr Asn Ile Tyr Arg Ala Leu Lys Cys Arg Glu
        255                 260                 265

cca gag agc aag gag ctc ctg tcc tac atg aga att cta tac aat atc 988
Pro Glu Ser Lys Glu Leu Leu Ser Tyr Met Arg Ile Leu Tyr Asn Ile
    270                 275                 280

tgc cct tac ttc aag ttt ggc tat atg gca gcc aat ggg gcg att gca 1036
Cys Pro Tyr Phe Lys Phe Gly Tyr Met Ala Ala Asn Gly Ala Ile Ala
285                 290                 295                 300

gaa gca ttg aga act gag aac aat atc cac ata att gat ttt cag att 1084
Glu Ala Leu Arg Thr Glu Asn Asn Ile His Ile Ile Asp Phe Gln Ile
                305                 310                 315

gct caa ggg act caa tgg atc aca ctg ata caa gca tta gct gca agg 1132
Ala Gln Gly Thr Gln Trp Ile Thr Leu Ile Gln Ala Leu Ala Ala Arg
            320                 325                 330

cct ggt ggt cct cct cgt gtg cgg atc acc ggg ata gat gac cca gtg 1180
Pro Gly Gly Pro Pro Arg Val Arg Ile Thr Gly Ile Asp Asp Pro Val
        335                 340                 345

tca gag tat gct cgt ggt gaa ggt ctt gac att gtg ggg aaa atg ttg 1228
Ser Glu Tyr Ala Arg Gly Glu Gly Leu Asp Ile Val Gly Lys Met Leu
    350                 355                 360

aaa agc atg tct gaa gaa ttc aaa ata cct ctg gag ttt acg cct ctg 1276
Lys Ser Met Ser Glu Glu Phe Lys Ile Pro Leu Glu Phe Thr Pro Leu
365                 370                 375                 380

tct gtc tat gcc aca caa gtc acg aaa gag atg ctt gag atc agg cca 1324

Ser Val Tyr Ala Thr Gln Val Thr Lys Glu Met Leu Glu Ile Arg Pro
                385                 390                 395

ggt gaa gca ctg tct gta aac ttc aca ctc cag cta cac cac acc ccg 1372
Gly Glu Ala Leu Ser Val Asn Phe Thr Leu Gln Leu His His Thr Pro
            400                 405                 410

gac gag agc gtg gat gtc aac aac cca cgc gat ggt ctg cta ccg gat 1420
Asp Glu Ser Val Asp Val Asn Asn Pro Arg Asp Gly Leu Leu Pro Asp
        415                 420                 425

ggt gaa agg gct gtc ccc gaa ggt gac tac ttt ggt aga gca gga gtc 1468
Gly Glu Arg Ala Val Pro Glu Gly Asp Tyr Phe Gly Arg Ala Gly Val
    430                 435                 440

aca cac caa cac aac gcc ttt ctt gat gag gtt tgg gga gac cat gga 1516
Thr His Gln His Asn Ala Phe Leu Asp Glu Val Trp Gly Asp His Gly
445                 450                 455                 460

gta cta ctc cgc cat gtt cga gtc gat cga ctg cca acc tgc cgc ggg 1564
Val Leu Leu Arg His Val Arg Val Asp Arg Leu Pro Thr Cys Arg Gly
                465                 470                 475

aca aca agg aga gga tca gcg tgg agc agc act gcc tcg cca agg cac 1612
Thr Thr Arg Arg Gly Ser Ala Trp Ser Ser Thr Ala Ser Pro Arg His
            480                 485                 490

atc gtc aac atc atc gcc tgc gag ggg aag gac agg gtg gag agg cat 1660

Ile Val Asn Ile Ile Ala Cys Glu Gly Lys Asp Arg Val Glu Arg His

        495                 500                 505

gag ctg ctt ggc aag tgg aag tcg agg ctg acc atg gcc ggc ttc agg 1708

Glu Leu Leu Gly Lys Trp Lys Ser Arg Leu Thr Met Ala Gly Phe Arg

    510                 515                 520

cct tac ccg ttg agc tcg tac gtc aac tcg gtg ata agg aag ctt ctc 1756

Pro Tyr Pro Leu Ser Ser Tyr Val Asn Ser Val Ile Arg Lys Leu Leu

525                 530                 535                 540

gcc tgc tac tcc gat aaa tac aca ttg gat gag aag gac ggc gcg atg 1804

Ala Cys Tyr Ser Asp Lys Tyr Thr Leu Asp Glu Lys Asp Gly Ala Met

                545                 550                 555

ctt ctc ggc tgg agg agc aga aag ctg ata tct gct tct gcg tgg cac 1852

Leu Leu Gly Trp Arg Ser Arg Lys Leu Ile Ser Ala Ser Ala Trp His

            560                 565                 570

tgacagtagt atgttgagaa gaagaaatga tagagagcat atatatacat accgattccc 1912

tggtccaatt tacagaatga taccctttga tgtaccctca gtttgattga cagcgggtgt 1972

tgccttcatt tatagaccag gatcaaacat tgacattaga tttgtagaga agttgtatga 2032

taataatttc tttgcgattt caatgtatga tgtatccatg gctgccatag ttaataatat 2092

tcttgcatag tcaaaaaaaa aaaaaaaaaa aaaaaaaaaa aaa 2135

<210> 2

<211> 572

<212> PRT

<213> Oryza sativa L cv Nipponbare

<400> 2

Met Asp Leu His Gln Leu Leu Lys Tyr Arg Leu Thr Gly Ala Asn Val
1               5                   10                  15

Val Tyr Glu Ile Pro Thr Glu Asn Asn Leu Gln Asn Ser Pro Trp Gln
            20                  25                  30

Ala Asn Pro Leu Lys Tyr Glu Phe Ser Asp Ser Pro Tyr Thr Pro Leu
        35                  40                  45

Ser Ser Gln Phe Glu Cys Asp Asn Leu Ser Ala Leu Thr Asn Thr Pro
    50                  55                  60

Asp Asn Gln Ser Ser Thr Glu Thr Ile Ser Ala Gln Pro Ile Ser Pro
65                  70                  75                  80

Leu Glu Ala Asp Ser Ser Tyr Arg Gln Ala Gly Ile Leu Leu Gln Glu
                85                  90                  95
Asn Ile Gln Val Gly Ala Asp Pro Leu Tyr Ala Thr Ser Arg His Asn
            100                 105                 110
Met Gln His Ala Leu Arg Glu Ile Glu Thr Val Leu Met Ala Pro Asp
        115                 120                 125
Thr Asp Asp Ala Thr Thr Ser Thr Lys His Glu Phe Glu Glu Ile Lys
    130                 135                 140
Pro Ala Gln Leu Val Arg Gln Arg Ser Arg Thr Trp Ser His Glu Ser
145                 150                 155                 160
Arg Gln Pro Leu Pro Gly Val Gly Arg Ser Gln Phe Ala Ser Gly Gly
                165                 170                 175
Tyr Pro Thr Ala Ser Tyr Glu Phe Arg Pro Glu Lys Arg Gln Arg Glu
            180                 185                 190
Leu Arg Glu Asp Pro Gln Ile Ile Val Lys Gln Leu Leu Thr Arg Cys
        195                 200                 205
Ala Glu Ala Leu Ser Glu Asp Arg Thr Glu Glu Phe His Lys Leu Val

    210                 215                 220

Gln Glu Ala Arg Gly Val Val Ser Ile Asn Gly Glu Pro Ile Gln Arg
225                 230                 235                 240

Leu Gly Ala Tyr Leu Leu Glu Gly Leu Val Ala Arg His Gly Asn Ser
                245                 250                 255

Gly Thr Asn Ile Tyr Arg Ala Leu Lys Cys Arg Glu Pro Glu Ser Lys
            260                 265                 270

Glu Leu Leu Ser Tyr Met Arg Ile Leu Tyr Asn Ile Cys Pro Tyr Phe
        275                 280                 285

Lys Phe Gly Tyr Met Ala Ala Asn Gly Ala Ile Ala Glu Ala Leu Arg
    290                 295                 300

Thr Glu Asn Asn Ile His Ile Ile Asp Phe Gln Ile Ala Gln Gly Thr
305                 310                 315                 320

Gln Trp Ile Thr Leu Ile Gln Ala Leu Ala Ala Arg Pro Gly Gly Pro
                325                 330                 335

Pro Arg Val Arg Ile Thr Gly Ile Asp Asp Pro Val Ser Glu Tyr Ala
            340                 345                 350

Arg Gly Glu Gly Leu Asp Ile Val Gly Lys Met Leu Lys Ser Met Ser
        355                 360                 365

Glu Glu Phe Lys Ile Pro Leu Glu Phe Thr Pro Leu Ser Val Tyr Ala
    370                 375                 380

Thr Gln Val Thr Lys Glu Met Leu Glu Ile Arg Pro Gly Glu Ala Leu
385                 390                 395                 400

Ser Val Asn Phe Thr Leu Gln Leu His His Thr Pro Asp Glu Ser Val
                405                 410                 415

Asp Val Asn Asn Pro Arg Asp Gly Leu Leu Pro Asp Gly Glu Arg Ala
            420                 425                 430

Val Pro Glu Gly Asp Tyr Phe Gly Arg Ala Gly Val Thr His Gln His
        435                 440                 445

Asn Ala Phe Leu Asp Glu Val Trp Gly Asp His Gly Val Leu Leu Arg
    450                 455                 460

His Val Arg Val Asp Arg Leu Pro Thr Cys Arg Gly Thr Thr Arg Arg
465                 470                 475                 480

Gly Ser Ala Trp Ser Ser Thr Ala Ser Pro Arg His Ile Val Asn Ile
                485                 490                 495

Ile Ala Cys Glu Gly Lys Asp Arg Val Glu Arg His Glu Leu Leu Gly
            500                 505                 510

Lys Trp Lys Ser Arg Leu Thr Met Ala Gly Phe Arg Pro Tyr Pro Leu
        515                 520                 525

Ser Ser Tyr Val Asn Ser Val Ile Arg Lys Leu Leu Ala Cys Tyr Ser
    530                 535                 540

Asp Lys Tyr Thr Leu Asp Glu Lys Asp Gly Ala Met Leu Leu Gly Trp
545                 550                 555                 560

Arg Ser Arg Lys Leu Ile Ser Ala Ser Ala Trp His
                565                 570

<210> 3
<211> 2204
<212> DNA
<213> Oryza sativa L cv Nipponbare

<220>
<221> CDS
<222> (301)..(1932)

<400> 3

cccgcgtccg cccacgcgtc cgcaccacct gcttcctccg cggctgcgtg cgccgcctcc 60

ccaacccttc gtcctccccg ccggccgccg ctgagatccg acgcgccgga gcggagctcg 120

ctgagatttt gttcctgctg gatttctgaa attttactgg aggactagag atatcaagta 180

caccaacaac aaactagtac aaagttttcc ttccagttaa tctgttcctg gatctccttg 240

ccatacgcga agcagtggat actatgcagt aattggttag gaggtcttta ctgaaccata 300

atg gct gat act cca act tcc cga atg att cat ccc ttc agc aat ata 348
Met Ala Asp Thr Pro Thr Ser Arg Met Ile His Pro Phe Ser Asn Ile
1               5                   10                  15

ccg agc caa aac ctg aag cag ttc cag tat tct gat aat cca cag cac 396
Pro Ser Gln Asn Leu Lys Gln Phe Gln Tyr Ser Asp Asn Pro Gln His
            20                  25                  30

cct tgc cac cct tat agg gca ccc tca gac acc cac gtt gtg cca cat 444
Pro Cys His Pro Tyr Arg Ala Pro Ser Asp Thr His Val Val Pro His
        35                  40                  45

cat tat ggc cta aag tca cat tca ccg gat gct ggt tat gaa agc cag 492
His Tyr Gly Leu Lys Ser His Ser Pro Asp Ala Gly Tyr Glu Ser Gln
    50                  55                  60

gct act cct aac aag tac aca ttg gac tcc tct gaa ggt gct ggt tgt 540
Ala Thr Pro Asn Lys Tyr Thr Leu Asp Ser Ser Glu Gly Ala Gly Cys
65                  70                  75                  80

atg aga cat gat tct cct tcc agt caa agt ttc aca acc aga agt ggt 588
Met Arg His Asp Ser Pro Ser Ser Gln Ser Phe Thr Thr Arg Ser Gly
                85                  90                  95

agc cct cta tct cag gaa gac agc cac tct gac tcg act gat gga tct 636
Ser Pro Leu Ser Gln Glu Asp Ser His Ser Asp Ser Thr Asp Gly Ser
            100                 105                 110

cct gta ggc gcc tcc tgt gtc act gag gat cct aat gat ctg aag caa 684
Pro Val Gly Ala Ser Cys Val Thr Glu Asp Pro Asn Asp Leu Lys Gln
        115                 120                 125

aaa ctg aag gat ctc gag gcc gtt atg ctt ggg cca gac tcg gaa ata 732
Lys Leu Lys Asp Leu Glu Ala Val Met Leu Gly Pro Asp Ser Glu Ile
    130                 135                 140

gtc aac agc ctt gag aat tcg gtg gca aac caa ctt tcc ttg gag ccg 780
Val Asn Ser Leu Glu Asn Ser Val Ala Asn Gln Leu Ser Leu Glu Pro
145                 150                 155                 160

gag aag tgg gtg cgc atg atg ggc att cct aga ggc aac ttg aaa gag 828

Glu Lys Trp Val Arg Met Met Gly Ile Pro Arg Gly Asn Leu Lys Glu
                165                 170                 175

cta ctg att gcc tgt gct aga gct gtg gaa gag aag aat agc ttt gct 876
Leu Leu Ile Ala Cys Ala Arg Ala Val Glu Glu Lys Asn Ser Phe Ala
            180                 185                 190

att gat atg atg att cca gag ctg aga aaa ata gtt tcg gta tct ggt 924
Ile Asp Met Met Ile Pro Glu Leu Arg Lys Ile Val Ser Val Ser Gly
        195                 200                 205

gag cca ctt gag agg ttg gga gcc tac atg gta gaa ggg ctt gtt gcc 972
Glu Pro Leu Glu Arg Leu Gly Ala Tyr Met Val Glu Gly Leu Val Ala
    210                 215                 220

agg ctt gcc tcc tct ggc att tca atc tac aaa gct ttg aaa tgc aag 1020
Arg Leu Ala Ser Ser Gly Ile Ser Ile Tyr Lys Ala Leu Lys Cys Lys
225                 230                 235                 240

gaa cca aag agc tct gac ctt ctg tct tac atg cac ttc ctg tat gag 1068
Glu Pro Lys Ser Ser Asp Leu Leu Ser Tyr Met His Phe Leu Tyr Glu
                245                 250                 255

gcc tgt ccc tac ttc aag ttt ggg tat atg tcg gca aat ggt gct att 1116
Ala Cys Pro Tyr Phe Lys Phe Gly Tyr Met Ser Ala Asn Gly Ala Ile
            260                 265                 270

gca gag gct gtc aag gga gaa gac agg att cat atc atc gac ttc cat 1164
Ala Glu Ala Val Lys Gly Glu Asp Arg Ile His Ile Ile Asp Phe His
        275                 280                 285

atc tct caa ggg gct cag tgg atc tct ctc ctt cag gcc ctt gca gcc 1212
Ile Ser Gln Gly Ala Gln Trp Ile Ser Leu Leu Gln Ala Leu Ala Ala
    290                 295                 300

agg cct ggt gga cca cca act gta agg atc act ggt att gat gac tca 1260
Arg Pro Gly Gly Pro Pro Thr Val Arg Ile Thr Gly Ile Asp Asp Ser
305                 310                 315                 320

gtg tca gct tac gca cga ggc ggc ggg cta gag ctg gtc gga agg agg 1308
Val Ser Ala Tyr Ala Arg Gly Gly Gly Leu Glu Leu Val Gly Arg Arg
                325                 330                 335

ctg tcg cac atc gcc agc ctg tgc aag gtg ccc ttt gaa ttc cac cca 1356
Leu Ser His Ile Ala Ser Leu Cys Lys Val Pro Phe Glu Phe His Pro
            340                 345                 350

ctt gct atc tcc ggc agc aag gtg gag gca gca cat ctc gga gtc atc 1404
Leu Ala Ile Ser Gly Ser Lys Val Glu Ala Ala His Leu Gly Val Ile
        355                 360                 365

ccc ggg gaa gcc ctc gcc gtg aac ttc acc ctg gag ctg cac cac att 1452

Pro Gly Glu Ala Leu Ala Val Asn Phe Thr Leu Glu Leu His His Ile
    370                 375                 380

cca gac gag tcg gtg agc acg gcg aac cac cgt gac cgc ctc cta agg 1500
Pro Asp Glu Ser Val Ser Thr Ala Asn His Arg Asp Arg Leu Leu Arg
385                 390                 395                 400

atg gtg aag agc ctg tcg ccg aag gta ctc acc ctg gtg gag atg gag 1548
Met Val Lys Ser Leu Ser Pro Lys Val Leu Thr Leu Val Glu Met Glu
                405                 410                 415

tcc aac acg aac acg gcg cca ttc ccg cag agg ttc gcg gag acg ctg 1596
Ser Asn Thr Asn Thr Ala Pro Phe Pro Gln Arg Phe Ala Glu Thr Leu
            420                 425                 430

gac tac tac acc gcc atc ttc gag tcc atc gac ctg acg ctg ccg agg 1644
Asp Tyr Tyr Thr Ala Ile Phe Glu Ser Ile Asp Leu Thr Leu Pro Arg
        435                 440                 445

gac gac agg gag agg atc aac atg gag cag cac tgc ctc gcg agg gag 1692
Asp Asp Arg Glu Arg Ile Asn Met Glu Gln His Cys Leu Ala Arg Glu
    450                 455                 460

atc gtc aac ctc atc gcg tgc gag ggg gag gag agg gcg gag cgg tac 1740
Ile Val Asn Leu Ile Ala Cys Glu Gly Glu Glu Arg Ala Glu Arg Tyr
465                 470                 475                 480

gag ccc ttc ggc aag tgg aag gcg cgc ctc acc atg gcc ggg ttc agg 1788
Glu Pro Phe Gly Lys Trp Lys Ala Arg Leu Thr Met Ala Gly Phe Arg
                485                 490                 495

ccg tcg ccg ctc agc tcg ctg gtg aac gcc acc atc agg acg ctg ctg 1836
Pro Ser Pro Leu Ser Ser Leu Val Asn Ala Thr Ile Arg Thr Leu Leu
            500                 505                 510

cag agc tac tcg gac aac tac aag ctc gcc gag agg gat ggg gcg ctc 1884
Gln Ser Tyr Ser Asp Asn Tyr Lys Leu Ala Glu Arg Asp Gly Ala Leu
        515                 520                 525

tac ctc ggg tgg aag agc agg ccc ctg gtg gtc tcg tcg gcg tgg cac 1932
Tyr Leu Gly Trp Lys Ser Arg Pro Leu Val Val Ser Ser Ala Trp His
    530                 535                 540

tagcgttgtg actttgtgag gtgatctgac gacgaacttc ccttgtaacc atcttggctg 1992

ggggctttag aaagctatag tgatcatgga ctggtaaggg tagctgtaac aatggtgttg 2052

taatggtaag gtancggcta gggtcaaact cgaaaatggg caaaatgctg atnaggtgta 2112

naagtgtagt acatgtttna nagtatatgt ttcagttttt cttttaaaaa aaaaaaaaaa 2172

aaaaaaaaaa aaaaaaaaaa aaaaaaaaaa aa 2204

<210> 4

<211> 544

<212> PRT

<213> Oryza sativa cv Nipponbare

<400> 4

Met Ala Asp Thr Pro Thr Ser Arg Met Ile His Pro Phe Ser Asn Ile

1               5                   10                  15

Pro Ser Gln Asn Leu Lys Gln Phe Gln Tyr Ser Asp Asn Pro Gln His

            20                  25                  30

Pro Cys His Pro Tyr Arg Ala Pro Ser Asp Thr His Val Val Pro His

        35                  40                  45

His Tyr Gly Leu Lys Ser His Ser Pro Asp Ala Gly Tyr Glu Ser Gln

    50                  55                  60

Ala Thr Pro Asn Lys Tyr Thr Leu Asp Ser Ser Glu Gly Ala Gly Cys

65                  70                  75                  80

Met Arg His Asp Ser Pro Ser Ser Gln Ser Phe Thr Thr Arg Ser Gly

                85                  90                  95

Ser Pro Leu Ser Gln Glu Asp Ser His Ser Asp Ser Thr Asp Gly Ser
            100                 105                 110
Pro Val Gly Ala Ser Cys Val Thr Glu Asp Pro Asn Asp Leu Lys Gln
        115                 120                 125
Lys Leu Lys Asp Leu Glu Ala Val Met Leu Gly Pro Asp Ser Glu Ile
    130                 135                 140
Val Asn Ser Leu Glu Asn Ser Val Ala Asn Gln Leu Ser Leu Glu Pro
145                 150                 155                 160
Glu Lys Trp Val Arg Met Met Gly Ile Pro Arg Gly Asn Leu Lys Glu
                165                 170                 175
Leu Leu Ile Ala Cys Ala Arg Ala Val Glu Glu Lys Asn Ser Phe Ala
            180                 185                 190
Ile Asp Met Met Ile Pro Glu Leu Arg Lys Ile Val Ser Val Ser Gly
        195                 200                 205
Glu Pro Leu Glu Arg Leu Gly Ala Tyr Met Val Glu Gly Leu Val Ala
    210                 215                 220
Arg Leu Ala Ser Ser Gly Ile Ser Ile Tyr Lys Ala Leu Lys Cys Lys
225                 230                 235                 240

Glu Pro Lys Ser Ser Asp Leu Leu Ser Tyr Met His Phe Leu Tyr Glu
                245                 250                 255

Ala Cys Pro Tyr Phe Lys Phe Gly Tyr Met Ser Ala Asn Gly Ala Ile
            260                 265                 270

Ala Glu Ala Val Lys Gly Glu Asp Arg Ile His Ile Ile Asp Phe His
        275                 280                 285

Ile Ser Gln Gly Ala Gln Trp Ile Ser Leu Leu Gln Ala Leu Ala Ala
    290                 295                 300

Arg Pro Gly Gly Pro Pro Thr Val Arg Ile Thr Gly Ile Asp Asp Ser
305                 310                 315                 320

Val Ser Ala Tyr Ala Arg Gly Gly Gly Leu Glu Leu Val Gly Arg Arg
                325                 330                 335

Leu Ser His Ile Ala Ser Leu Cys Lys Val Pro Phe Glu Phe His Pro
            340                 345                 350

Leu Ala Ile Ser Gly Ser Lys Val Glu Ala Ala His Leu Gly Val Ile
        355                 360                 365

Pro Gly Glu Ala Leu Ala Val Asn Phe Thr Leu Glu Leu His His Ile

370                    375                    380

Pro Asp Glu Ser Val Ser Thr Ala Asn His Arg Asp Arg Leu Leu Arg
385                    390                    395                    400

Met Val Lys Ser Leu Ser Pro Lys Val Leu Thr Leu Val Glu Met Glu
                    405                    410                    415

Ser Asn Thr Asn Thr Ala Pro Phe Pro Gln Arg Phe Ala Glu Thr Leu
               420                    425                    430

Asp Tyr Tyr Thr Ala Ile Phe Glu Ser Ile Asp Leu Thr Leu Pro Arg
          435                    440                    445

Asp Asp Arg Glu Arg Ile Asn Met Glu Gln His Cys Leu Ala Arg Glu
     450                    455                    460

Ile Val Asn Leu Ile Ala Cys Glu Gly Glu Glu Arg Ala Glu Arg Tyr
465                    470                    475                    480

Glu Pro Phe Gly Lys Trp Lys Ala Arg Leu Thr Met Ala Gly Phe Arg
                    485                    490                    495

Pro Ser Pro Leu Ser Ser Leu Val Asn Ala Thr Ile Arg Thr Leu Leu
               500                    505                    510

Gln Ser Tyr Ser Asp Asn Tyr Lys Leu Ala Glu Arg Asp Gly Ala Leu
        515                 520                 525

Tyr Leu Gly Trp Lys Ser Arg Pro Leu Val Val Ser Ser Ala Trp His
    530                 535                 540

<210> 5
<211> 625
<212> PRT
<213> Oryza sativa L cv Nipponbare

<400> 5
Met Lys Arg Glu Tyr Gln Glu Ala Gly Gly Ser Ser Gly Gly Gly Ser
  1               5                  10                  15

Ser Ala Asp Met Gly Ser Cys Lys Asp Lys Val Met Ala Gly Ala Ala
             20                  25                  30

Gly Glu Glu Glu Asp Val Asp Glu Leu Leu Ala Ala Leu Gly Tyr Lys
         35                  40                  45

Val Arg Ser Ser Asp Met Ala Asp Val Ala Gln Lys Leu Glu Gln Leu
     50                  55                  60

Glu Met Ala Met Gly Met Gly Gly Val Ser Ala Pro Gly Ala Ala Asp

65                  70                  75                  80

Asp Gly Phe Val Ser His Leu Ala Thr Asp Thr Val His Tyr Asn Pro

                85                  90                  95

Ser Asp Leu Ser Ser Trp Val Glu Ser Met Leu Ser Glu Leu Asn Ala

            100                 105                 110

Pro Leu Pro Pro Ile Pro Pro Ala Pro Pro Ala Ala Arg His Ala Ser

        115                 120                 125

Thr Ser Ser Thr Val Thr Gly Gly Gly Gly Ser Gly Phe Phe Glu Leu

    130                 135                 140

Pro Ala Ala Ala Asp Ser Ser Ser Ser Thr Tyr Ala Leu Arg Pro Ile

145                 150                 155                 160

Ser Leu Pro Val Val Ala Thr Ala Asp Pro Ser Ala Ala Asp Ser Ala

                165                 170                 175

Arg Asp Thr Lys Arg Met Arg Thr Gly Gly Gly Ser Thr Ser Ser Ser

            180                 185                 190

Ser Ser Ser Ser Ser Ser Leu Gly Gly Gly Ala Ser Arg Gly Ser Val

        195                 200                 205

Val Glu Ala Ala Pro Pro Ala Thr Gln Gly Ala Ala Ala Ala Asn Ala
    210                 215                 220
Pro Ala Val Pro Val Val Val Val Asp Thr Gln Glu Ala Gly Ile Arg
225                 230                 235                 240
Leu Val His Ala Leu Leu Ala Cys Ala Glu Ala Val Gln Gln Glu Asn
                245                 250                 255
Phe Ala Ala Ala Glu Ala Leu Val Lys Gln Ile Pro Thr Leu Ala Ala
            260                 265                 270
Ser Gln Gly Gly Ala Met Arg Lys Val Ala Ala Tyr Phe Gly Glu Ala
        275                 280                 285
Leu Ala Arg Arg Val Tyr Arg Phe Arg Pro Ala Asp Ser Thr Leu Leu
    290                 295                 300
Asp Ala Ala Phe Ala Asp Leu Leu His Ala His Phe Tyr Glu Ser Cys
305                 310                 315                 320
Pro Tyr Leu Lys Phe Ala His Phe Thr Ala Asn Gln Ala Ile Leu Glu
                325                 330                 335
Ala Phe Ala Gly Cys His Arg Val His Val Val Asp Phe Gly Ile Lys
            340                 345                 350

Gln Gly Met Gln Trp Pro Ala Leu Leu Gln Ala Leu Ala Leu Arg Pro
        355                 360                 365
Gly Gly Pro Pro Ser Phe Arg Leu Thr Gly Val Gly Pro Pro Gln Pro
    370                 375                 380
Asp Glu Thr Asp Ala Leu Gln Gln Val Gly Trp Lys Leu Ala Gln Phe
385                 390                 395                 400
Ala His Thr Ile Arg Val Asp Phe Gln Tyr Arg Gly Leu Val Ala Ala
                405                 410                 415
Thr Leu Ala Asp Leu Glu Pro Phe Met Leu Gln Pro Glu Gly Glu Ala
            420                 425                 430
Asp Ala Asn Glu Glu Pro Glu Val Ile Ala Val Asn Ser Val Phe Glu
        435                 440                 445
Leu His Arg Leu Leu Ala Gln Pro Gly Ala Leu Glu Lys Val Leu Gly
    450                 455                 460
Thr Val His Ala Val Arg Pro Arg Ile Val Thr Val Val Glu Gln Glu
465                 470                 475                 480
Ala Asn His Asn Ser Gly Ser Phe Leu Asp Arg Phe Thr Glu Ser Leu

                485                 490                 495

His Tyr Tyr Ser Thr Met Phe Asp Ser Leu Glu Gly Gly Ser Ser Gly

            500                 505                 510

Gln Ala Glu Leu Ser Pro Pro Ala Ala Gly Gly Gly Gly Gly Thr Asp

        515                 520                 525

Gln Val Met Ser Glu Val Tyr Leu Gly Arg Gln Ile Cys Asn Val Val

    530                 535                 540

Ala Cys Glu Gly Ala Glu Arg Thr Glu Arg His Glu Thr Leu Gly Gln

545                 550                 555                 560

Trp Arg Asn Arg Leu Gly Arg Ala Gly Phe Glu Pro Val His Leu Gly

                565                 570                 575

Ser Asn Ala Tyr Lys Gln Ala Ser Thr Leu Leu Ala Leu Phe Ala Gly

            580                 585                 590

Gly Asp Gly Tyr Arg Val Glu Glu Lys Glu Gly Cys Leu Thr Leu Gly

        595                 600                 605

Trp His Thr Arg Pro Leu Ile Ala Thr Ser Ala Trp Arg Val Ala Ala

    610                 615                 620

Ala

625

<210> 6

<211> 427

<212> PRT

<213> Lycopersicon esculentum

<400> 6

Leu Gly Ser Phe Gly Ser Ser Ser Ser Gln Ser His Pro His His Asp

1               5                   10                  15

Glu Glu Ser Ser Asp His His Gln Gln Arg Arg Phe Thr Ala Thr Ala

            20                  25                  30

Thr Thr Ile Thr Thr Thr Thr Ile Thr Thr Ser Pro Ala Ile Gln Ile

        35                  40                  45

Arg Gln Leu Leu Ile Ser Cys Ala Glu Leu Ile Ser Gln Ser Asp Phe

    50                  55                  60

Ser Ala Ala Lys Arg Leu Leu Thr Ile Leu Ser Thr Asn Ser Ser Pro

65                  70                  75                  80

Phe Gly Asp Ser Thr Glu Arg Leu Val His Gln Phe Thr Arg Ala Leu
                85                  90                  95
Ser Leu Arg Leu Asn Arg Tyr Ile Ser Ser Thr Thr Asn His Phe Met
            100                 105                 110
Thr Pro Val Glu Thr Thr Pro Thr Asp Ser Ser Ser Ser Ser Ser Leu
        115                 120                 125
Ala Leu Ile Gln Ser Ser Tyr Leu Ser Leu Asn Gln Val Thr Pro Phe
    130                 135                 140
Ile Arg Phe Thr Gln Leu Thr Ala Asn Gln Ala Ile Leu Glu Ala Ile
145                 150                 155                 160
Asn Gly Asn His Gln Ala Ile His Ile Val Asp Phe Asp Ile Asn His
                165                 170                 175
Gly Val Gln Trp Pro Pro Leu Met Gln Ala Leu Ala Asp Arg Tyr Pro
            180                 185                 190
Ala Pro Thr Leu Arg Ile Thr Gly Thr Gly Asn Asp Leu Asp Thr Leu
        195                 200                 205
Arg Arg Thr Gly Asp Arg Leu Ala Lys Phe Ala His Ser Leu Gly Leu
    210                 215                 220

Arg Phe Gln Phe His Pro Leu Tyr Ile Ala Asn Asn Asn His Asp His
225                 230                 235                 240

Asp Glu Asp Pro Ser Ile Ile Ser Ser Ile Val Leu Leu Pro Asp Glu
                245                 250                 255

Thr Leu Ala Ile Asn Cys Val Phe Tyr Leu His Arg Leu Leu Lys Asp
            260                 265                 270

Arg Glu Lys Leu Arg Ile Phe Leu His Arg Val Lys Ser Met Asn Pro
        275                 280                 285

Lys Ile Val Thr Ile Ala Glu Lys Glu Ala Asn His Asn His Pro Leu
    290                 295                 300

Phe Leu Gln Arg Phe Ile Glu Ala Leu Asp Tyr Tyr Thr Ala Val Phe
305                 310                 315                 320

Asp Ser Leu Glu Ala Thr Leu Pro Pro Gly Ser Arg Glu Arg Met Thr
                325                 330                 335

Val Glu Gln Val Trp Phe Gly Arg Glu Ile Val Asp Ile Val Ala Met
            340                 345                 350

Glu Gly Asp Lys Arg Lys Glu Arg His Glu Arg Phe Arg Ser Trp Glu

        355                 360                 365

Val Met Leu Arg Ser Cys Gly Phe Ser Asn Val Ala Leu Ser Pro Phe

    370                 375                 380

Ala Leu Ser Gln Ala Lys Leu Leu Leu Arg Leu His Tyr Pro Ser Glu

385                 390                 395                 400

Gly Tyr Gln Leu Gly Val Ser Ser Asn Ser Phe Phe Leu Gly Trp Gln

                405                 410                 415

Asn Gln Pro Leu Phe Ser Ile Ser Ser Trp Arg

            420                 425

## Description

### Technical Field

The present invention relates to plant elicitor- and gibberellin-responsive genes, and uses thereof.

### Background Art

DiLaurenzio *et al*. used chromosome walking to isolate and elucidate the structure of the SCARECROW gene, which controls the radial organization of Arabidopsis roots and stems (Laurenzio, L., D., Wysocka-Diller, J., Malamy, J. E., Pysh, L., Helariutta, Y., Freshour, G., Hahn, M. G., Feldmann, K. A., and Benfey, P. N., "The SCARECROW gene regulated an asymmetric cell division that is essential for generating the radial organization of the Arabidopsis root." Cell, 1996, 86, 423-433). The SCARECROW gene was presumed from its structure to be a transcription factor. A number of genes with structures similar to the SCARECROW gene were identified in the Arabidopsis EST, showing clearly that SCARECROW formed a gene family (Pysh, L. D., Wysocka-Diller, J. W., Camilleri, C., Bouchez, D., and Benfey, P. N., "The GRAS gene family in Arabidopsis: sequence characterization and basic expression analysis of the SCARECROW-LIKE genes" The Plant J., 1999, 18, 111-119).

Independently to this, two genes (GAI and RGA) that negatively regulate the signals of "gibberellin", a plant hormone, were isolated in Arabidopsis. The predicted amino acid sequences displayed significant homology with SCARECROW, especially the two-thirds at the C-terminal end (Peng, J., Carol, P., Richards, D. E., King, K. E., Cowling, R. J., Murphy, G. P., and Harberd, N. P., "Genes and Development" 1997, 11, 3194-3205; Silverstone, A. L., Ciampaglio, C. N., and Sun, T-P., "The Arabidopsis RGA gene encodes a transcriptional regulator repressing the gibberellin signal transduction pathway." The Plant Cell, 1998, 10, 155-169).

Similar gibberellin signal regulatory factors were also isolated in corn, wheat (Peng, J., Richards, D. E., Hartley, N. M., Murphy, G. P., Devos, K. M., Flintham, J. E., Beales, J., Fish, L. J., Worland, A. J., Pelica, F., Sudhakar, D., Christou, P., Snape, J. W., Gale, M. D., and Harberd, N. P., "'Green revolution' genes encode mutant gibberellin response modulators." Nature, 1999, 400, 256-261), and rice (Ogawa, M., Kusano, T., Katsumi, M., and Sano, H., "Rice gibberellin-insensitive gene homolog, OsGAI, encodes a nuclear-localized protein capable of gene activation at transcriptional level." Gene, 2000, 245, 21-29; Ikeda, A., Ueguchi-Tanaka, M. , Sonoda, Y. , Kitano, H. , Koshioka, M. , Futsuhara, Y., Matsuoka, M., and Yamaguchi, J:, "slender rice, a constitutive gibberellin response mutant, is caused by a null mutation of the SLR1 gene, an ortholog of the height-regulating gene GAI/RGA/RHT/D8." The Plant Cell, 2001, 13, 999-1010), indicating structural conservation. In addition, the Lateral suppressor gene product in tomatoes was reported as belonging to the same family as SCARECROW (Schumacher, K., Schmitt, T., Rossberg, M., Schmitz, G., and Theres, K., "The Lateral suppressor(Ls) gene of tomato encodes a new member of the VHIID protein family." Proc. Natl. Acad. Sci. U.S.A., 1999, 96, 290-295).

Pysh *et al*. named this gene family GRAS, taking the capital letters of GAI, RGA and SCARECROW, which were found in Arabidopsis (Pysh, L. D., Wysocka-Diller, J. W., Camilleri, C., Bouchez, D., and Benfey, P. N., "The GRAS gene family in Arabidopsis: sequence characterization and basic expression analysis of the SCARECROW-LIKE genes." The Plant J., 1999, 18, 111-119). The amino acid sequences of the GRAS family are characterized by, for example, low homology in the N-terminal region, a leucine heptad structure, and VHIID regions. This type of structure is characteristic to plants, and is not known in animals or microorganisms.

Gibberellin signal regulatory factors share high homology upto the N-terminal. In particular, genetic research on GAI has revealed that the DELLA region plays a significant role in catching the gibberellin signal (Herbard, N. P., King, K. , E. , Carol, P. , Cowling, R. J., Peng, J., and Richards, D. E. BioEssays, 1998, 20, 1001-1008).

The SCARECROW amino acid sequence includes the basic amino acid region found in leucine zipper-type transcription factors. Since 44% of the 267 amino acid residues at the N-terminal are glutamic acid, serine, threonine, proline and the like, SCARECROW was presumed to be a transcription factor (Laurenzio, L., D., Wysocka-Diller, J., Malamy, J. E., Pysh, L., Helariutta, Y., Freshour, G., Hahn, M. G., Feldmann, K. A., and Benfey, P. N., "The SCARECROW gene regulated an asymmetric cell division that is essential for generating the radial organization of the Arabidopsis root." Cell, 1996, 86, 423-433).

Recently, the nuclear localization of fluorescence in recombinants introduced with RGA-GFP fusion proteins suggested that RGA was a transcription factor localized to nuclei (Silverstone, A. L. , Ciampaglio, C. N. , and Sun, T-P. , "The Arabidopsis RGA gene encodes a transcriptional regulator repressing the gibberellin signal transduction pathway." The Plant Cell, 1998, 10, 155-169).

Chitin oligomers (N-acetylchitooligosaccharides), major components of the cell wall of rice blast disease fungus were found to induce various defense responses in cultured rice cells at low concentrations. In other words, these substances were found to act as powerful elicitors (substances that induce biological defense responses) (Yamada, A., Shibuya, N., Kodama, O., and Akatsuka, T., "Induction of phytoalexin formation in suspension-cultured rice cells by N-acetylchitooligosaccharides", Biosci. Biotech. Biochem. (1993) 57, 405-409). In this process of chitin oligomer action, known genes of defense-related enzymes, PAL, chitinase, and glucanase, were shown to be expressed (He, D.-Y., Yazaki, Y., Nishizawa, Y., Takai, R., Yamada, K., Sakano, K., Shibuya, N., and Minami, E., "Gene activation by cytoplasmic acidification in suspension-cultured rice cells in response to the potent elicitor, N-acetylchitoheptaose", Mol. Plant-Microbe Int. (1998) 12, 1167-1174; Nishizawa, Y., Kawakami, A., Hibi, T., He, D.-Y., Shibuya, N., and Minami, E., "Regulation of the chitinase gene expression in suspension-cultured rice cells by N-acetylchitooligosaccharides: differences in the signal transduction pathways leading to the activation of elicitor-responsive genes", Plant Mol. Biol. (1999) 39, 907-914). In addition, three novel early genes, EL2, EL3, and EL5, have been found to be expressed with more rapid time-courses (Minami, E., Kuchitsu, K., He, D.-Y., Kouchi, H., Midoh, N., Ohtsuki, Y., and Shibuya, N., "Two novel genes rapidly and transiently activated in suspension-cultured rice cells by treatment with N-acetylchitoheptaose, a biotic elicitor for phytoalexin production", Plant Cell Physiol. (1996) 37, 563-567; Takai, R. , Hasegawa, K. , Kaku, K. , Shibuya, N. , and Minami, E. , "Isolation and analysis of expression mechanisms of a rice gene, EL5, which shows structural similarity to ATL family from Arabidopsis, in response to N-acetylchitooligosaccharide elicitor", Plant Sci. (2001) 160, 577-583), and their structures and expression characteristics were revealed.

### Disclosure of the Invention

An object of the present invention is to identify novel elicitor-responsive genes in plants, and to provide these genes and plants in which these genes are regulated.

To achieve the above tasks, the present inventors conducted exhaustive studies. First, since the expression of a number of genes is thought to change in response to elicitors, the present inventors used a DNA microchip, containing 1265 varieties of rice EST, to screen for genes whose expression is induced in the early stages of elicitor treatment. A chitin oligomer (N-acetylchitooligosaccharide), an important component in the cell wall of rice blast fungus, was used as the elicitor. As a result, six types of novel elicitor-responsive ESTs, including two types with SCARECROW family cDNA, were identified. Of these, the products of the two SCARECROW-like genes (named CIGR1 and CIGR2 genes) had GRAS family-specific motifs, revealing for the first time that GRAS family members, other than Gibberellin signal transduction regulatory factors, also exist in rice. Interestingly, in a rice suspension cell culture and the absence of auxin, the expression of these two genes was induced in response to gibberellin in a very short period. Gibberellin is the generic name for compounds that comprise the *ent*-gibberellane backbone. However, only active forms of gibberellin are effective in inducing the expression of these genes. In addition, the signal transduction process leading to the expression of these genes suggests the involvement of protein phosphorylation and dephosphorylation. This kind of sudden gene expression in response to gibberellin was also observed in rice green leaves.

In plants, elicitors are known to induce various defense-related enzyme genes, and to bring about a defense response. Thus, it is expected that the elicitor-inducible CIGR1 and CIGR2 genes will be of value in conferring disease resistance to crops. Gibberellin is used as an agricultural hormone for governing essential crop characteristics, such as dormancy or budding. However, a gibberellin-inducible transcription factor has yet to be reported. Thus, it is anticipated that the genes of the present invention will also be useful in the production of recombinant crops whose valuable characteristics are regulated by gibberellin.

Thus, the present invention relates to elicitor- and gibberellin-responsive genes, and to uses thereof. Specifically, the present invention relates to:
[1] a DNA encoding a plant protein, wherein the DNA is any one of (a) to (d):
   (a) a DNA comprising the nucleotide sequence of SEQ ID NO: 1 or SEQ ID NO: 3;
   (b) a DNA that hybridizes under stringent conditions with the DNA comprising the nucleotide sequence of SEQ ID NO: 1 or SEQ ID NO: 3;
   (c) a DNA comprising the amino acid sequence of SEQ ID NO: 2 or SEQ ID NO: 4;
   (d) a DNA comprising the amino acid sequence of SEQ ID NO: 2 or SEQ ID NO: 4, in which one or more amino acids have been substituted, deleted, added, and/or inserted;
[2] the DNA of [1], wherein the plant is rice;
[3] a DNA encoding a protein that comprises a characteristic dominant negative for the protein coded by the DNA of [1] or [2];
[4] a protein coded by a DNA of any one of [1] to [3];
[5] a nucleic acid of any one of (a) to (d):
   (a) an antisense nucleic acid complementary to the transcription product of the DNA of [1] or [2];
   (b) a nucleic acid comprising the ribozyme activity of specifically cleaving the transcription product of the DNA of [1] or [2];
   (c) a nucleic acid comprising the effect of inhibiting the expression of the DNA of [1] or [2] by co-suppression;
   (d) a nucleic acid comprising the effect of inhibiting the expression of the DNA of [1] or [2] by an RNAi effect;
[6] a vector comprising the nucleic acid of [5] or a DNA of any one of [1] to [3];
[7] a transformed plant cell that maintains a DNA of any one of [1] to [3], the nucleic acid of [5], or the vector of [6];
[8] a transformed plant comprising the transformed plant cell of [7];
[9] the transformed plant of [8], wherein the plant is rice-derived;
[10] a transformed plant that is an offspring or clone of the transformed plant of [8] or [9];
[11] a reproductive material of a transformed plant of any one of [8] to [10];
[12] a method for producing a transformed plant of any one of [8] to [10], which comprises the step of inserting a DNA of any one of [1] to [3], the nucleic acid of [5], or the vector of [6] into a plant cell to reproduce a plant from the plant cell;
[13] the method of [12], wherein the plant is rice.

The present inventors identified two novel elicitor-responsive genes in plants: the CIGR1 and CIGR2 genes. They also revealed that these genes display responsiveness to gibberellin.

The present invention provides DNAs coding for plant CIGR2 and CIGR1 proteins. Examples of the above-mentioned plant of the present invention include, without limitation, valuable agricultural products such as cereals, vegetables and fruit trees, and ornamental plants such as foliage plants, etc. Specifically, examples of these plants can include rice, corn, wheat, barley, rapeseed, soybeans, cotton, tomatoes, potatoes, chrysanthemums, roses, carnations, cyclamens, and such.

The above-mentioned plant of the present invention preferably includes rice. The nucleotide sequence of the cDNA of the rice CIGR1 gene is presented in SEQ ID NO: 1, and SEQ ID NO: 2 shows the amino acid sequence of the protein coded by this cDNA. Equally, the CIGR2 gene's cDNA sequence is shown in SEQ ID NO: 3, and the amino acid sequence of the protein coded by this cDNA is presented in SEQ ID NO: 4.

Where it occurs in plants other than rice, the DNA of the present invention can be isolated using methods typically known to one skilled in the art. These methods include, for example, methods using hybridization technique (Southern, EM., J. Mol. Biol., 1975, 98, 503) or Polymerase Chain Reaction (PCR) technique (Saiki, RK. *et al*., Science, 1985, 230, 1350; Saiki, RK. *et al*., Science, 1988, 239, 487). Thus, DNA with high homology to a'DNA that comprises SEQ ID NO: 1 or 3 can generally be isolated from another plant by one skilled in the art. This can be achieved by using, as a probe, a DNA comprising SEQ ID NO: 1 or 3, or one part of this DNA, and using as primers, oligonucleotides that specifically hybridize to a DNA comprising SEQ ID NO: 1 or 3. Thus, a DNA of the present invention also includes DNA that hybridizes to a DNA comprising SEQ ID NO: 1 or 3, isolated using hybridization or PCR technology in this way.

To isolate this kind of DNA, a hybridization reaction is preferably carried out under stringent conditions. Stringent hybridization conditions in the present invention refer to 6 M urea, 0.4% SDS and 0.5 x SSC, or to stringent hybridization conditions similar to these. Further stringent conditions, (for example, 6 M urea, 0.4% SDS and 0.1 x SSC) are expected to facilitate the isolation of DNA with greater homology. The DNA isolated in this way is thought to have high homology at the amino acid level with the amino acid sequence of SEQ ID NO: 2 or 4. "High homology" with the full length amino acid sequence means sequence identity of at least 70% or more, preferably 80% or more, further preferably 90% or more, and most preferably 95% or more.

Homology in amino acid or nucleotide sequences can be determined using Karlin and Altschul's BLAST algorithm (Proc. Natl. Acad. Sei. USA, 1990, 87, 2264-2268; Karlin, S. & Altschul, SF., Proc. Natl. Acad. Sei. USA, 1993, 90, 5873). Programs called BLASTN and BLASTX have been developed using the BLAST algorithm as a base (Altschul, SF. *et al*., J. Mol. Biol., 1990, 215, 403). When using BLASTN to analyze nucleotide sequences, the parameters can be set at, for example, score=100 and word length=12. In addition, when using BLASTX to analyze amino acid sequences, the parameters can be set at, for example, score=50 and word length=3. When using BLAST and the Gapped BLAST program, the default parameters for each program are used. Specific techniques for these analysis methods are in the well known (http://www.ncbi.nlm.nih.gov/).

The present invention also provides DNA that codes for proteins with a structural resemblance to the above plant CIGR1 and CIGR2 proteins. This DNA includes DNA coding a protein that comprises the amino acid sequence of the CIGR1 or CIGR2 protein, where one or more amino acid substitutions, deletions, additions and/or insertions have been made.

In preparing the above DNA, one skilled in the art could use well known methods, such as the above-mentioned hybridization technique (Southern, EM., J. Mol. Biol., 1975, 98, 503)or PCR technique (Saiki, RK. *et al*., Science, 1985, 230, 1350; Saiki, RK. *et al*., Science, 1988, 239, 487). In addition to these methods, techniques such as, for example, introducing mutations using site-directed mutagenesis (Kramer, W. & Fritz, HJ. , Methods Enzymol., 1987, 154, 350) are also included. In the natural world, mutations in the nucleotide sequence can cause mutations in the amino acid sequence of the coded protein. In some cases, a nucleotide sequence mutation will not lead to a mutation in the amino acid sequence of the protein (a degenerate mutation). Such degenerately mutated DNAs are also included in the present invention.

The DNA of the present invention includes genomic DNA, cDNA and chemically synthesized DNA. Genomic DNA and cDNA can be produced by practices well used by those skilled in the art. For example, genomic DNA can be prepared as follows: Genomic DNA is extracted from plants comprising genes coding for the above-mentioned plant CIGR1 and CIGR2 proteins, a genomic library is constructed (a plasmid, phage, cosmid, BAG, PAC or the like can be used as the vector) and developed, and colony or plaque hybridization can be carried out using probes prepared by using a DNA coding for an above-mentioned protein as a base. Alternatively, genomic DNA can also be prepared by constructing primers specific to a DNA coding for an above-mentioned plant CIGR1 or CIGR2 protein, and then using these primers to carry out PCR. cDNA can be prepared, for example, as follows: cDNA is synthesized based on mRNA extracted from plants which comprise a gene coding for one of the above-mentioned proteins, this cDNA is inserted into λZAP vector or such, a cDNA library is prepared and developed, and cDNA can then be prepared in the same way as above, by carrying out colony or phage hybridization, or PCR.

The DNA of the above invention can be used, for example, in the preparation of recombinant proteins, and in the production of transformant plants whose phenotype has been altered by regulating DNA expression.

A recombinant protein is usually prepared by inserting a DNA of the present invention into an appropriate expression vector, introducing the vector into an appropriate cell, culturing the transformed cells, and purifying the expressed proteins. A recombinant protein can be expressed as a fusion protein with other proteins to make purification easier, for example, as a fusion protein with maltose binding protein in *Escherichia coli* (New England Biolabs, USA, vector pMAL series), as a fusion protein with glutathione-S-transferase (GST) (Amersham Pharmacia Biotech, vector pGEX series), or tagged with histidine (Novagen, pET series). The host cell is not limited so long as the cell is suitable for expressing the recombinant protein. It is possible to utilize, for example, yeast, plant, insect cells or various other animal cells, besides the above-described E. coli, by altering the expression vector used. A vector can be introduced into a host cell by a variety of methods known to one skilled in the art. For example, a transformation method using calcium ions (Mandel, M. and Higa, A., Journal of Molecular Biology, 1970, 53, 158-162; Hanahan, D. , Journal of Molecular Biology, 1983, 166, 557-580) can be used to introduce the vector into E. coli. A recombinant protein expressed in the host cells can be purified and recovered from the host cells or the culture supernatant thereof by known methods in the art. When a recombinant protein is expressed as a fusion protein with maltose binding protein or other partners, the recombinant protein can be easily purified via affinity chromatography. Thus generated proteins encoded by the DNAs of the present invention are also included in the present invention.

The resulting protein can be used to prepare an antibody that binds to the protein. For example, a polyclonal antibody can be prepared from serum by immunizing animals used for immunizations, such as rabbits, with a purified protein of the present invention or a portion thereof, collecting blood after a certain period, and using serum in which clots have been removed. A monoclonal antibody can be prepared by fusing myeloma cells with the antibody-forming cells of animals immunized with the above protein or its portion, isolating a monoclonal cell expressing a desired antibody (hybridoma) , and recovering the antibody from the cell. The antibody thus obtained can be utilized to purify or detect a protein of the present invention. The antibodies of the present invention include antisera, polyclonal antibodies, monoclonal antibodies, and fragments thereof.

A plant transformant expressing a DNA of the present invention can be created by inserting the DNA of the present invention into an appropriate vector, introducing this vector into a plant cell, and then, regenerating the resulting transformed plant cell.

Furthermore, a plant transformant can be created in which the expression of a DNA of the present invention is suppressed. This is achieved using a DNA that suppresses the expression of a DNA of the present invention, wherein the DNA is inserted into an appropriate vector, the vector is introduced into a plant cell, and the resulting transformed plant cell is regenerated. The phrase "suppression of expression of a DNA of the present invention" includes suppression of gene transcription and/or suppression of translation to a protein. Furthermore, it also includes a complete inability to express DNA, as well as reduced expression.

Examples of preferable embodiments of a DNA used to inhibit the expression of a DNA of the present invention may include antisense nucleic acids complementary to the transcription product of a DNA of this invention, nucleic acids comprising the ribozyme activity of specifically cleaving a transcription product of a DNA of this invention, nucleic acids which inhibit the expression of a DNA of this invention by the effects of RNAi or co-suppression, and a DNA coding for a protein which comprises a characteristic that is dominant-negative for a transcription product of a DNA of this invention, and such. In the present invention, "nucleic acid" means RNA or DNA.

The expression of a specific endogenous gene in plants can be suppressed by methods utilizing antisense technology conventional to the art. Ecker *et al*. were the first to demonstrate the antisense effect of an antisense RNA introduced by electroporation into plant cells (J. R. Ecker and R. W. Davis, Proc. Natl. Acad. Sci. USA, 1986, 83, 5372). Thereafter, target gene expression was reportedly reduced in tobacco and petunias by expressing antisense nucleic acids (A. R. van der Krol *et al*., Nature, 1988, 333, 866). The antisense technique has now been established as a means of suppressing target-gene expression in plants.

Multiple factors cause an antisense nucleic acid to suppress target-gene expression. These include the following: inhibition of transcription initiation by triple strand formation; suppression of transcription by hybrid formation at the site where the RNA polymerase has formed a local open loop structure; transcription inhibition by hybrid formation with the RNA being synthesized; suppression of splicing by hybrid formation at the junction between an intron and an exon; suppression of splicing by hybrid formation at the site of spliceosome formation; suppression of mRNA translocation from the nucleus to the cytoplasm by hybrid formation with mRNA; suppression of splicing by hybrid formation at the capping site or at the poly (A) addition site; suppression of translation initiation by hybrid formation at the binding site for the translation initiation factors; suppression of translation by hybrid formation at the site for ribosome binding near the initiation codon; inhibition of peptide chain elongation by hybrid formation in the translated region or at the polysome binding sites of mRNA; and suppression of gene expression by hybrid formation at the sites of interaction between nucleic acids and proteins. These antisense nucleic acids suppress target gene expression by inhibiting the process of transcription, splicing, translation, or such (Hirashima and Inoue, "Shin Seikagaku Jikken Koza (New Biochemistry Experimentation Lectures) 2, Kakusan (Nucleic Acids) IV, Idenshi No Fukusei To Hatsugen (Replication and Expression of Genes) ," Nihon Seikagakukai Hen (The Japanese Biochemical Society) , Tokyo Kagaku Dozin, 1993, pp. 319-347).

An antisense nucleic acid of the present invention can suppress target gene expression by any of the above mechanisms. In one embodiment, if an antisense sequence is designed to be complementary to the untranslated region near the 5' end of a gene's mRNA, it will effectively inhibit translation of the gene. It is also possible to use sequences complementary to the coding regions or to the untranslated region on the 3' side. Thus, the antisense nucleic acid used in the present invention includes a nucleic acid having antisense sequences against both the untranslated regions and the translated regions of the gene. An antisense nucleic acid to be used is connected downstream of an appropriate promoter, and, preferably, a sequence containing the transcription termination signal is connected on the 3' side. The nucleic acid thus prepared can be transfected into the desired plant by known methods. The sequence of the antisense nucleic acid is preferably a sequence complementary to the endogenous gene of the plant to be transformed or a part thereof, but it need not be perfectly complementary so long as it can effectively inhibit gene expression. The transcribed RNA is preferably 90% or more, and most preferably 95% or more complementary to the transcribed products of the target gene. In order to effectively inhibit the expression of the target gene by means of an antisense nucleic acid, the antisense nucleic acid should be at least 15 nucleotides long or more, preferably 100 nucleotides long or more, and still more preferably 500 nucleotides long or more. The antisense nucleic acid to be used is generally shorter than 5 kb, and preferably shorter than 2.5 kb.

Furthermore, DNA encoding ribozymes can also be used to suppress the expression of endogenous genes. A ribozyme means an RNA molecule with catalytic activities. There are many ribozymes having various activities. Research with a focus on ribozymes as RNA-cleaving enzymes has enabled the design of a ribozyme that site-specifically cleaves RNA. While some ribozymes of the group I intron type or the M1RNA contained in RNaseP consist of 400 nucleotides or more, others belonging to the hammerhead type or the hairpin type have an activity domain of about 40 nucleotides (Makoto Koizumi and Eiko Ohtsuka, Tanpakushitsu Kakusan Kohso (Nucleic acid, Protein, and Enzyme) , 1990, 35, 2191).

The self-cleavage domain of a hammerhead type ribozyme cleaves at the 3' side of C15 in the sequence G13U14C15. Formation of a nucleotide pair between U14 and A at the ninth position is considered important for the ribozyme activity. It has been shown that the cleavage also occurs when the nucleotide at the 15th position is A or U instead of C (M. Koizumi *et al*. FEBS Lett., 1988, 228, 228). If the substrate binding site of the ribozyme is designed to be complementary to the RNA sequences adjacent to the target site, one can create a restriction-enzyme-like RNA-cleaving ribozyme which recognizes the sequence UC, UU, or UA within the target RNA (M. Koizumi *et al*. FEBS Lett., 1988, 239, 285; Makoto Koizumi and Eiko Ohtsuka, Tanpakushitsu Kakusan Kohso (Protein, Nucleic acid, and Enzyme), 1990, 35, 2191; M. Koizumi *et al*. Nucleic Acids Res. , 1989, 17, 7059). For example, in the coding region of the CIGR1 or CIGR2 gene, there are pluralities of sites that can be used as the ribozyme target.

Hairpin-type ribozymes are also useful in the present invention. These ribozymes can be found, for example, in the minus strand of tobacco ringspot virus satellite RNA (J. M. Buzayan, Nature,1986, 323, 349). The hairpin-type ribozyme has also been shown to create RNA-cleaving ribozymes that specifically target RNA (Y. Kikuchi and N. Sasaki, Nucleic Acids Res., 1991, 19, 6751; Yo Kikuchi, Kagaku To Seibutsu (Chemistry and Biology), 1992, 30, 112).

A ribozyme designed to cleave a target is fused with a promoter, such as the cauliflower mosaic virus 35S promoter, and with a transcription termination sequence, so that it will be transcribed in plant cells. If extra sequences are added to the 5' end or the 3' end of the transcribed RNA, the ribozyme activity can be lost. In this case, one can place an additional trimming ribozyme, which functions in cis on the 5' or the 3' side of the ribozyme portion, in order to precisely cut the ribozyme portion from the transcribed RNA containing the ribozyme (K. Taira *et al*., Protein Eng., 1990, 3, 733; A. M. Dzaianott and J. J. Bujarski, Proc. Natl. Acad. Sci. USA, 1989, 86, 4823; C. A. Grosshands and R. T. Cech, Nucleic Acids Res., 1991, 19, 3875; K. Taira *et al*., Nucleic Acid Res., 1991, 19, 5125). Multiple sites within the target gene can be cleaved by arranging these structural units in tandem to achieve greater effect (N. Yuyama *et al*., Biochem. Biophys. Res. Commun. , 1992, 186, 1271) . By using such ribozymes, it is possible to specifically cleave the transcription products of a target gene in the present invention, thereby suppressing expression of that gene.

Inhibition of endogenous gene expression can also be carried out by RNAi (RNA interference) , which uses double-stranded RNA (dsRNA) comprising a sequence resembling or identical to a target gene sequence. "RNAi" refers to the phenomenon where the expression of introduced exogenous genes as well as target endogenous genes is inhibited upon introducing cells with dsRNA comprising a sequence which resembles or is identical to the target gene sequence. The details of the RNAi mechanism are not clear, however it is thought that the initially introduced dsRNA is degraded into small pieces, and somehow becomes an indicator of the target gene, resulting in target gene degradation. RNAi is also known to have effect in plants (Chuang, CF. & Meyerowitz, EM., Proc. Natl. Acad. Sci. USA, 2000, 97, 4985). For example, to inhibit the expression of plant gene CIGR1 or CIGR2 using RNAi, dsRNA comprising the CIGR1 or CIGR2 gene sequence, or a sequence resembling one of these genes, can be inserted into a plant. Genes used as RNAi do not need to be completely identical to the target gene, however they comprise at least 70% or more, preferably 80% or more, more preferably 90% or more, and most preferably 95% or more identity. Sequence identity can be determined using an above-mentioned method.

Inhibition of endogenous gene expression can also be achieved by co-suppression, caused by a DNA transformant which comprises a sequence identical or similar to a target gene sequence. "Co-suppression" refers to a phenomenon where the expressions of an introduced exogenous gene and a target endogenous gene are inhibited upon using a transformant to introduce plants with a gene which comprises a sequence identical or similar to the target endogenous gene. The details of the co-suppression mechanism are not clear, however at least one part is thought to duplicate the RNAi mechanism. Co-suppression is also observed in plants (Smyth, DR., Curr Biol, 1997, 7, R793; Martienssen, R. , Curr Biol, 1996, 6, 810). For example, to produce plants in which the CIGR1 or CIGR2 gene has been co-suppressed, the target plant can be transformed with vector DNA constructed to enable the expression of a DNA which comprises the CIGR1 or CIGR2 gene sequence, or a sequence resembling one of these genes. Genes for use in co-suppression are not required to be completely identical to the target gene, however comprise at least 70% or more, preferably 80% or more, more preferably 90% or more, and most preferably 95% or more identity. The degree of sequence identity can be determined using an above-described method.

Expression inhibition of an endogenous gene of the present invention can be achieved by transforming a plant with a gene which encodes a protein comprising characteristics dominant-negative to a protein coding for the target gene. "A gene which codes a protein comprising dominant-negative characteristics" refers to a gene which, when expressed, comprises the function of eliminating or reducing the activity of the plant's original endogenous wild type protein.

In addition, the present invention provides the above-mentioned DNAs, vectors comprising the nucleic acids, transformed plant cells comprising the vectors, transformed plants comprising the transformed plant cells, transformed plants that are offspring or clones of the above transformed plants, and breeding materials from the transformed plants.

Moreover, the present invention provides a method for producing the above-mentioned transformed plants that includes the process of introducing a DNA or nucleic acid of the present invention or a vector of the present invention into plant cells, and regenerating plants from the plant cells.

A DNA or nucleic acid of the present invention can be introduced into plant cells by one skilled in the art using known methods such as the agrobacterium method, electroporation method, and the particle gun method.

The method of Nagel *et al*., for example, is used for the agrobacterium method (Microbiol. Lett., 1990, 67, 325). According to this method, agrobacterium is transformed by a recombinant vector and introduced to plant cells using a known method such as the leaf disc method. The above vector comprises, for example, a promoter to express a DNA of the present invention in a plant subsequent to introduction into that plant. Generally, a DNA of the present invention is placed downstream of such a promoter and, moreover, a terminator sequence is placed downstream of the DNA. A recombinant vector used for this purpose is suitably determined by one skilled in the art, depending on the transfection method or type of plant. The above-mentioned promoter may be, for example, a cauliflower mosaic virus derived CaMV35S promoter or the ubiquitin promoter from maize (Unexamined Published Japanese Patent Application No. (JP-A) Hei 2-79983).

The above-mentioned terminator may be, for example, a cauliflower mosaic virus-derived terminator or nopalin synthase terminator. However, so long as they function as a promoter or terminator in a plant, they are not limited.

Plants transfected by a DNA or nucleic acid of the present invention may be explants. Alternatively, cultured cells may be prepared from these plants, and such nucleic acids may be introduced into the cultured cells. "Plant cells" in the present invention may be, for example, cells from leaves, roots, stems, flowers, seed scutella, calluses, and cultured cell suspensions.

In addition, to efficiently select transformed plant cells which have been introduced with a DNA or nucleic acid of the present invention, the above recombinant vector preferably harbors an appropriate selective marker gene, or is introduced into plant cells together with a plasmid vector harboring a selective marker gene. Selective marker genes used for this purpose include, for example, the hygromycin phosphotransferase gene, which confers resistance to the antibiotic hygromycin; the neomycin phosphotransferase gene, which confers resistance to kanamycin or gentamycin; and the acetyltransferase gene, which confers resistance to the herbicide, phosphinothricin.

Plant cells transfected with a recombinant vector are plated and cultured on a known selective medium containing an appropriate selective drug, depending on the type of the introduced selective marker gene. In this way, one can obtain transformed plant cultured cells.

A plant is then regenerated from the transformed plant cells into which a DNA or nucleic acid of the present invention has been introduced. Regeneration of a plant can be carried out by methods known to one skilled in the art depending on the plant cell type (Toki *et al*., Plant Physiol., 1995, 100, 1503-1507). Several techniques have already been established to generate transformed rice plants, and those techniques are widely used in the field of the present invention. For example, rice plants can be regenerated after genes are introduced using (1) polyethylene glycol (suitable for Indica rice varieties)(Datta, S. K. *et al*., In Gene Transfer To Plants (Potrykus I and Spangenberg Eds.), 1995, 66-74) ; (2) electric pulse (suitable for Japonica rice varieties) (Toki *et al*., Plant Physiol. , 1992, 100, 1503-1507); (3) particle gun method (Christou *et al*., Bio/technology, 1991, 9, 957-962) ; or (4) agrobacteria (Hiei *et al*., Plant J. , 1994, 6, 271-282). In the present invention, these methods can preferably be used.

The plants regenerated from transformed plant cells are subsequently cultured in acclimatization medium. After the acclimatized regenerated plants are grown under normal cultivation conditions, plants can be obtained. Seeds can also be obtained when these plants mature and produce fruit.

The exogenously introduced DNA or nucleic acid in a thus regenerated and grown transformed plant can be confirmed by known methods, such as PCR or Southern hybridization, or by analyzing the nucleotide sequence of the plant's nucleic acid. To extract DNA or nucleic acid from a transformed plant, the known method of J. Sambrook *et al*. may be used (Molecular Cloning, 2^{nd} edition, Cold Spring Harbor laboratory Press, 1989).

To conduct PCR analysis of the exogenous gene comprising a DNA of the present invention, which exists in the regenerated plant body, an amplification reaction is carried out using the template nucleic acid that was extracted from the regenerated plant by the above-mentioned method. When the nucleic acid of the present invention is a DNA, the amplification reaction may be carried out in a reaction mixture containing, as primers, synthesized oligonucleotides comprising nucleotide sequences appropriately selected according to the DNA's nucleotide sequence. An amplified DNA fragment comprising a DNA sequence of the present invention may be obtained by repeating the denaturation, annealing, and extension steps for DNA several dozen cycles of the amplification reaction. The respective amplified DNA fragments can be separated by, for example, electrophoresing the reaction solution containing amplified products on agarose gel. DNA fragments corresponding to a DNA of the present invention can then be confirmed.

Having obtained a transformed plant in which a DNA of the present invention has been inserted into the chromosomes, one can obtain the plant's offspring by sexual or non-sexual reproduction. Also, it is possible to mass-produce such plants by obtaining reproductive materials (such as seeds, fruits, cuttings, stem tubers, root tubers, shoots, calluses, and protoplasts) from the above plant, or its offspring or clones.

### Brief Description of the Drawings

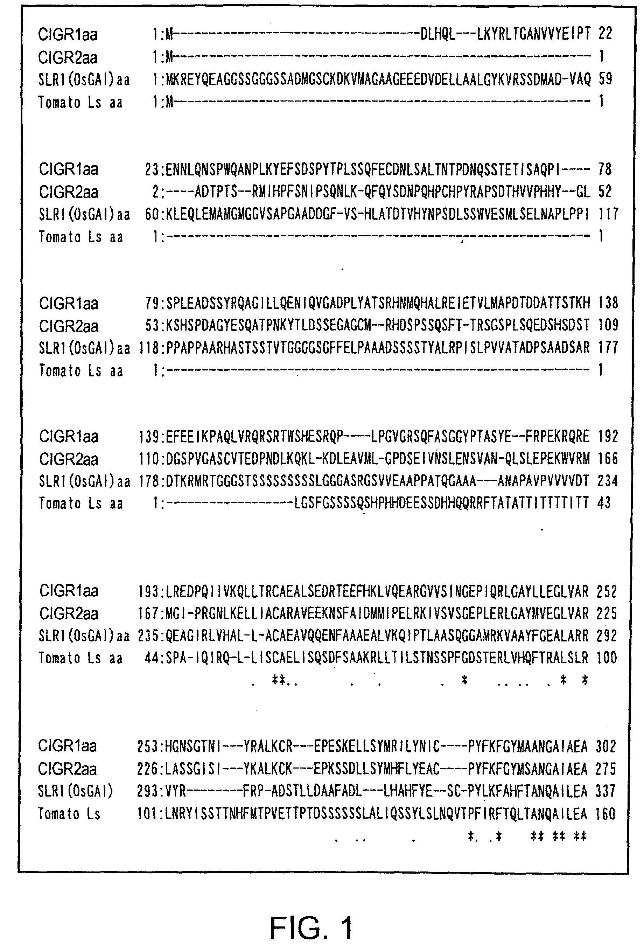

Fig. 1 depicts comparisons of the amino acid sequences encoded by CIGR1 gene and CIGR2 gene with those of the GRAS family. SLR1 (OsGAI) indicates the rice gibberellin signal repressor (SEQ ID NO: 5) and Tomato Ls indicates the tomato lateral bud suppressor (SEQ ID NO: 6). Amino acid residues conserved in all four sequences are indicated by *, and amino acid residues conserved in three sequences are indicated by ^{•}.

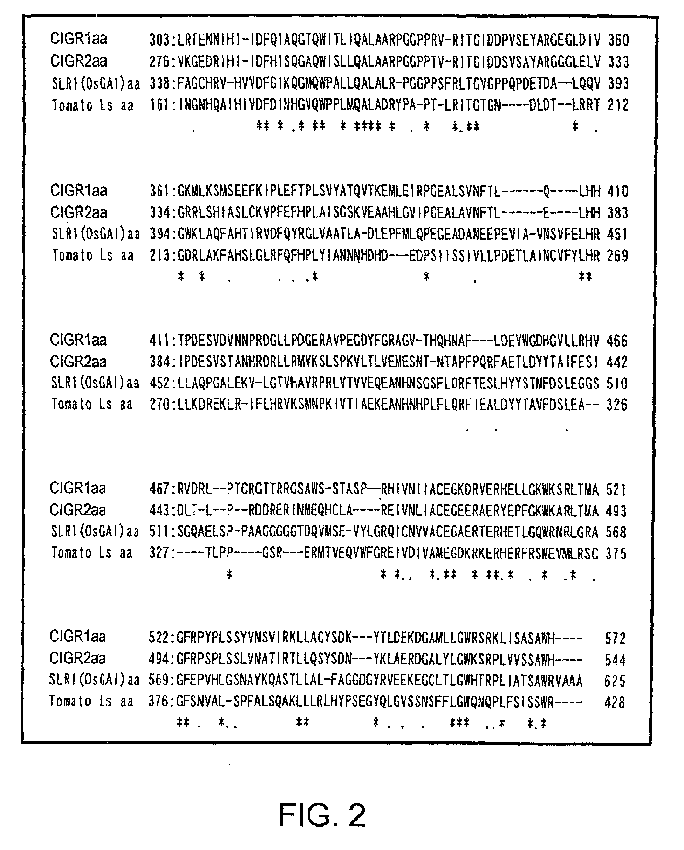

Fig. 2 is the continuation of Fig. 1.

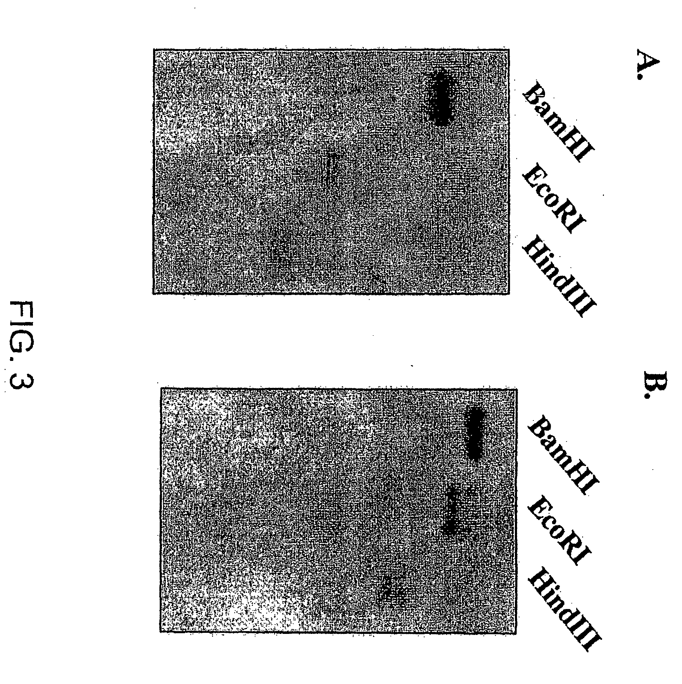

Fig. 3 depicts photographs representing the results of genomic Southern hybridization of CIGR1 gene and CIGR2 gene. A: CIGR1 gene; B: CIGR2 gene.

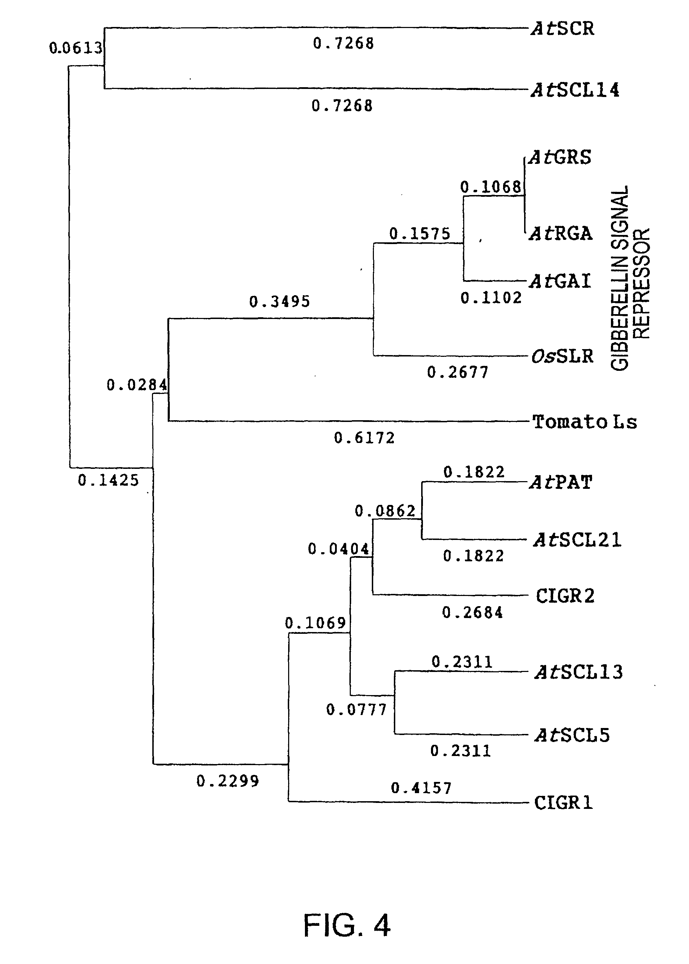

Fig. 4 shows the phylogenetic relationship of CIGR1 gene and CIGR2 gene at the amino acid level. AtSCR indicates the Arabidopsis SCARECROW. AtSCLn indicates SCARECROW-like genes. AtGRS indicates an AtGAI-like gene, whose function is unknown. AtGRA indicates an Arabidopsis gibberellin signal repressor. AtGAI indicates an Arabidopsis gibberellin signal repressor (functional share with GRA is unknown). OsSLR indicates the rice gibberellin signal repressor. Tomato Ls indicates the tomato lateral bud suppressor. AtPAT indicates the Arabidopsis light signal transduction factor. AtSCL21 indicates an Arabidopsis SCARECROW-like gene (function unknown). CIGR2 indicates a rice gene reported in this study. AtSCL13 indicates an Arabidopsis SCARECROW-like gene (function unknown). AtSCL5 indicates an Arabidopsis SCARECROW-like gene (function unknown). CIGR1 indicates a rice gene reported in this study.

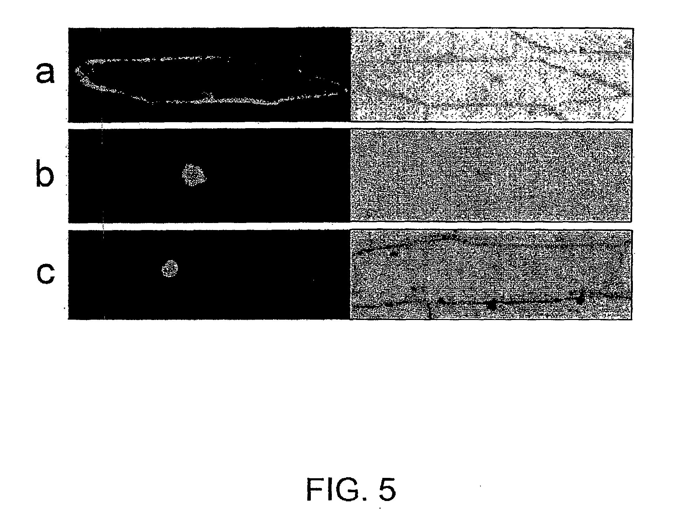

Fig. 5 depicts photographs representing the localization of CIGR1 and CIGR2 genes in the nucleus. The fused gene 35S/CIGR1/GFP or 35S/CIGR2/GFP was introduced into onion epidermal cells using the particle gun method. A laser confocal microscope was used for observation. The fused gene 35S/GFP was used as the control. a: 35S/GFP; b: 35S/CIGR1/GFP; c: 35S/CIGR2/GFP.

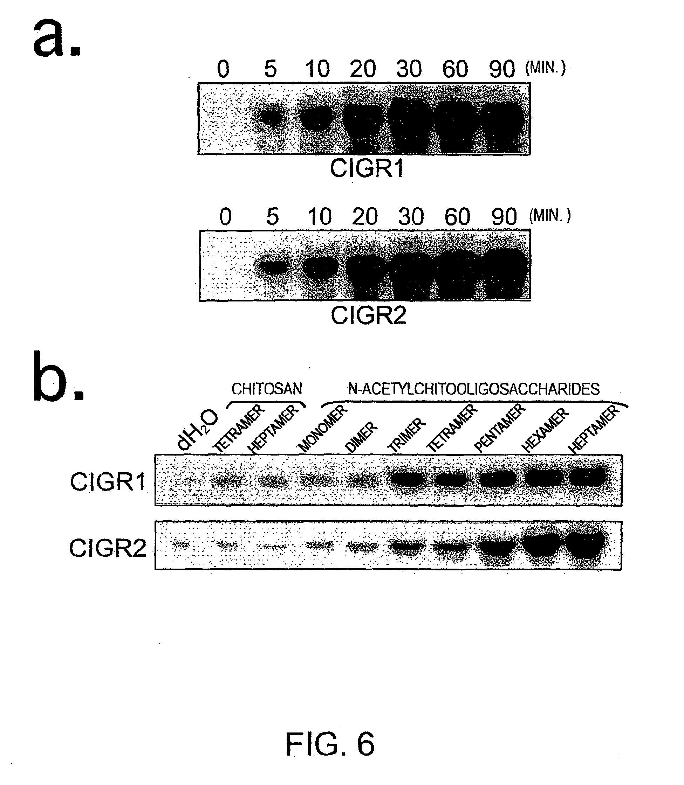

Fig. 6 depicts photographs representing the responsiveness of CIGR1 gene and CIGR2 gene to chitin oligomers. a: time-courses (min.) of expression induced by treatment with chitin heptamer. b: induction activities of the chitin and chitosan oligomers.

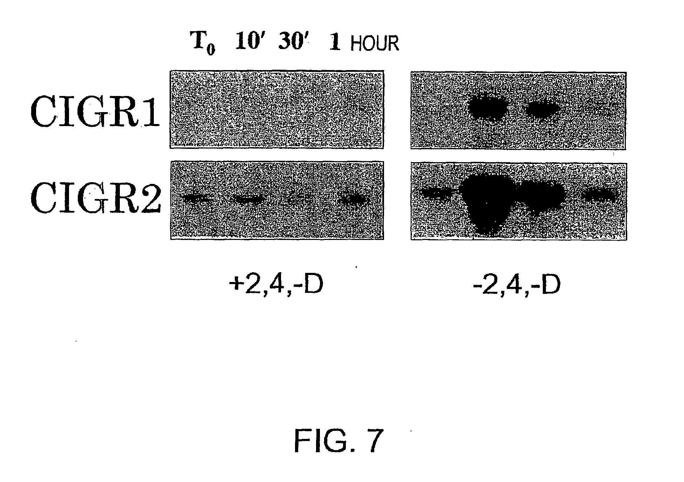

Fig. 7 depicts photographs representing the effect of 2,4-D on the responses of CIGR1 gene and CIGR2 gene to GA3. Time indicated is the time lapsed after the GA3 treatment.

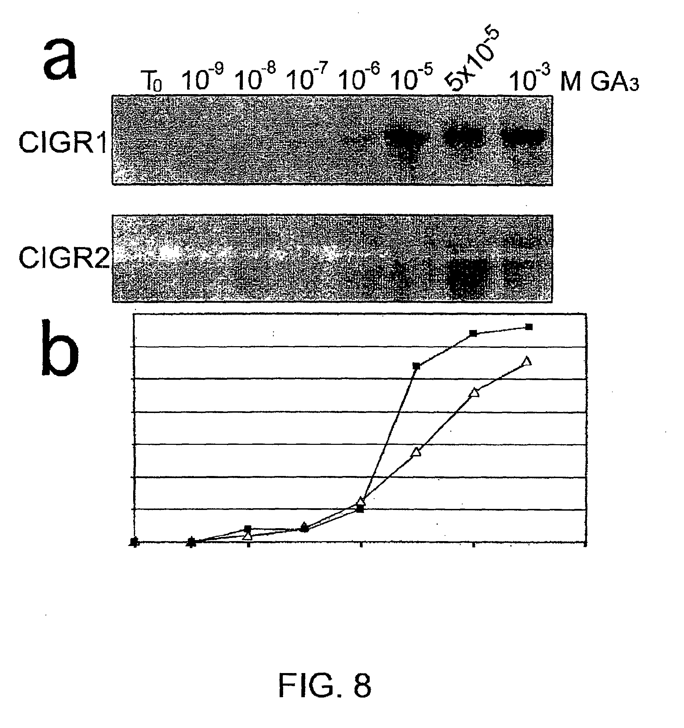

Fig. 8 depicts photographs and a diagram representing the effect of GA3 concentration on the induction of expression of CIGR1 gene and CIGR2 gene. a: photographs representing the results of Northern blot hybridization analysis of total RNA extracted after the GA3 treatment for ten minutes at various concentrations (expressed in M). b: diagram representing the results of quantitative determination of signals in 'a' by an image analyzer. Squares indicate CIGR1 gene and open triangles indicate CIGR2 gene.

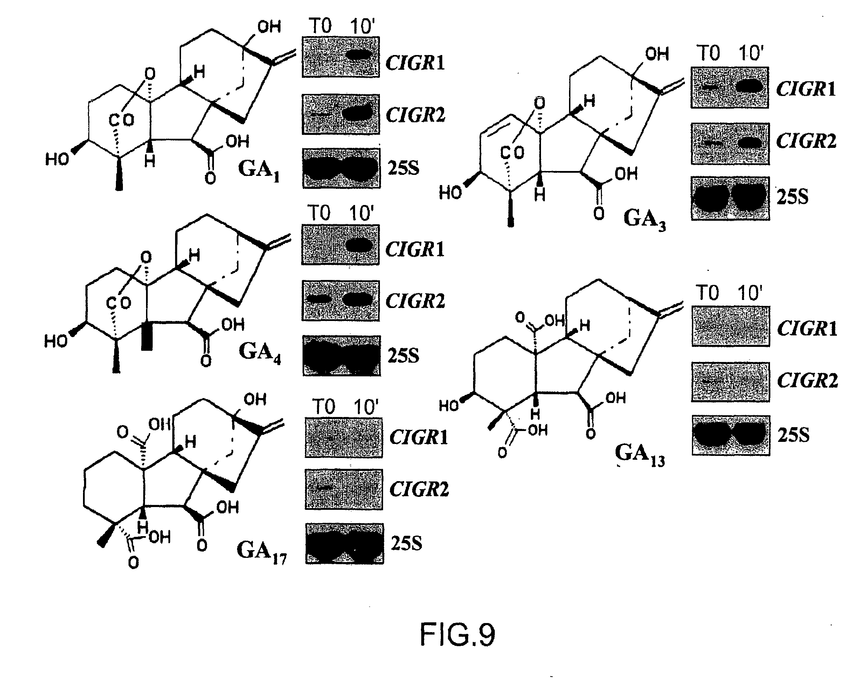

Fig. 9 depicts diagrams and photographs representing the physiological activities of gibberellins and expression of genes. Total RNA was extracted after a ten-minute treatment of cultured rice cells with active gibberellin species (GA1, GA3, or GA4) or inactive gibberellin species (GA13 or GA17). The photographs show the results of analysis by the Northern blot hybridization method.

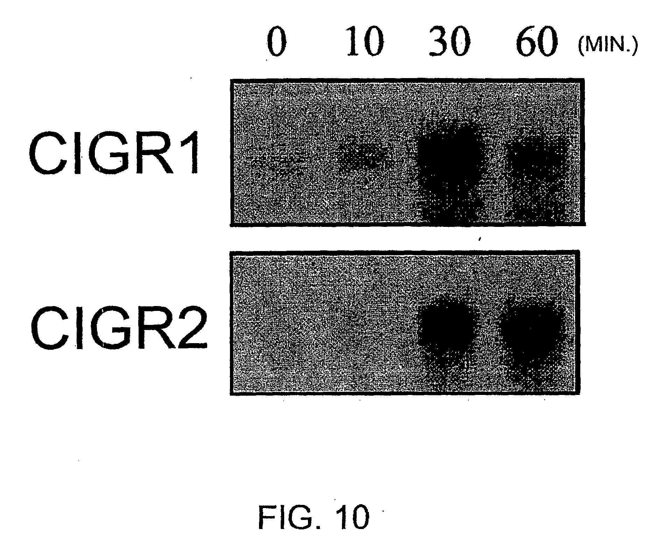

Fig. 10 depicts photographs representing the expression of CIGR1 gene and CIGR2 gene in green leaves of rice after GA3 treatment. After spraying rice plants with GA3, the third and fourth leaves were sampled at certain intervals. Total RNA extracted from the third and fourth leaves was analyzed by the Northern blot hybridization method.

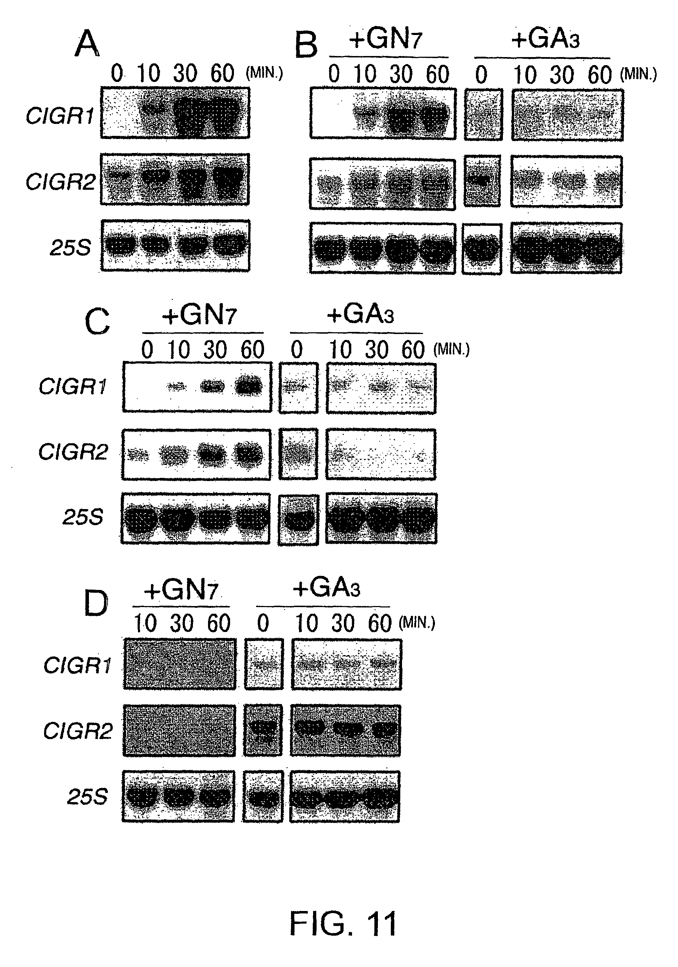

Fig. 11 depicts photographs representing the effects of protein kinase inhibitors on elicitor response and gibberellin response. The following compounds were added ten minutes before the treatment with chitin heptamer (GN7) or gibberellin (GA3). (A): the positive control of the elicitor response; (B): okadaic acid (1 µM); (C), lavendustin A (30 µM); (D), K-252a (20 µM).

### Best Mode for Carrying out the Invention

Herein below, the present invention will be specifically described with reference to Examples, but it is not to be construed as being limited thereto. The Examples were carried out using the materials and methods described below.
(1) Cultured rice cells: The cultured cells of rice were induced from germinating rice seeds (*Oryza sativa* cv. Nipponbare) on a N6 agar plate containing 1 ppm 2, 4-D (auxin; 2,4-dichlorophenoxyacetic acid). The cells were successively cultured in liquid N6 medium as follows. Once a week, cells of about 1 ml in volume were transferred to 150 ml N6 medium. Every two weeks, sizes of the clusters of cells were reduced by passing through a 20-mesh metallic mesh. Unfiltered cells that were cultured while shaking for four to six days after transferring to a 30-ml medium, were used for Northern blotting's RNA extraction .
(2) RNA extraction: The extraction of total RNA from cultured cells and green leaves of rice was carried out following the phenol-SDS method. A Polytron homogenizer was used to homogenize tissue in the presence of phenol (about 90% saturated with water) of about tenfold or more volume to the tissue volume, and the same volume of extraction buffer solution (50 mM Tris-HCl pH 9.0, 1% SDS, 50 mM NaCl). The aqueous phase obtained by centrifugation was repeatedly extracted with phenol. The total nucleic acid was precipitated by adding 0.6 volume of isopropanol to the extract and then stirring the mixture. After dissolving the precipitate in water, 0.25 volume of 10M LiCl was added, and the mixture was cooled on ice. High molecular weight RNA was collected by centrifugation, washed with 70% ethanol, and then dissolved in a small volume of water to be used as an RNA sample.
(3) A method for gibberellin treatment of cultured cells and green leaves: The cultured cells were pre-washed with 2,4-D-free N6 medium several times, and then suspended in the same medium and shaken for two hours at 25°C. Gibberellin (GA3 dissolved in ethanol) was added to make the respective final concentrations and the cells were cultured while shaking for a predetermined time and used for RNA extraction. Nipponbare plants, grown three weeks after seeding, were sprayed with gibberellin. The third and fourth leaves were collected at certain intervals and total RNA was extracted. Elicitor treatment was carried out by directly adding aqueous elicitor solutions to cultured cells of rice.
(4) Hybridization: RNA for the Northern hybridization was denatured by the glyoxal method. 2.7 µl of glyoxal (final concentration, 1M), 1.6 µl of sodium phosphate (pH 7.0, final concentration, 10 mM), and 8 µl of dimethylsulfoxide (final concentration, 50%) were added to 10 µg of total RNA (3.7 µl). The mixture was kept at 50°C for one hour and then subjected to electrophoresis in 1.4% agarose (10 mM sodium phosphate, pH 7.0). After electrophoresis, RNA was blotted onto a nylon membrane (Biodyne A) and RNA was fixed onto the membrane by treatment at 80°C for two hours. Hybridization was carried out in a mixture of 50% formamide, 0.1% SDS, 0.1 mg/ml salmon sperm DNA, 5 x SSPE (0.9 M NaCl, 50 mM sodium phosphate, 5 mM EDTA, pH 7.4), and 5 x Denhardt's solution (0.1% bovine serum albumin, 0.1% Ficoll, 0.1% polyvinylpyrrolidone) at 42°C for one day. The membrane was then washed with 0.1 x SSC (15 mM NaCl, 1.5 mM sodium citrate) three times for five minutes each at room temperature and twice for 30 minutes each at 65°C and then exposed on X-ray film.
(5) DNA microarray analysis: Microchip (http://cdna01.dna.affrc.go.jp/RMOS/index.html) was prepared using rice ESTs (1265 clones) by the Rice Genome Project. Single-stranded cDNA probes were prepared by the reverse transcription of poly (A) -RNA extracted from the cells with no elicitor treatment and with a 15-minute elicitor treatment, in the presence of cy5-dCTP. The result was analyzed using a Microarray Scanner FLA8000 (Fujifilm).

### [Example 1]

Of the rice GRAS family members, genes regarded as gibberellin signal repressors (SLR and OsGAI genes) were recently reported (Ogawa, M., Kusano, T., Katsumi, M., and Sano, H. "Rice gibberellin-insensitive gene homolog, OsGAI, encodes a nuclear-localized protein capable of gene activation at transcriptional level.", Gene, 2000, 245, 21-29; Ikeda, A., Ueguchi-Tanaka, M., Sonoda, Y., Kitano, H., Koshioka, M., Futsuhara, Y., Matsuoka, M., and Yamaguchi, J. "slender rice, a constitutive gibberellin response mutant, is caused by a null mutation of the SLR1 gene, an ortholog of the height-regulating gene GAI/RGA/RHT/D8.", The Plant Cell, 2001, 13, 999-1010). Two MAFF Genebank ESTs (c72495 and AU094860) that showed significantly increased signals on DNA microarrays after a 15-minute elicitor treatment were obtained from the Ministry of Agriculture, Forestry and Fisheries (MAFF) Genebank , and their entire nucleotide sequences were determined. Their deduced amino acid sequences were compared with the known sequences of GRAS family members. The search results indicated that these two sequences had homology with known GRAS family genes (Fig. 1 and Fig. 2). The two genes had VHIID regions and C-terminal amino acid sequences that were highly conserved between SCARECROW and its family. However, they had no typical leucine heptad structure. The DELLA sequence, which is characteristic of the gibberellin signal repressors, was not present. Hereafter, C72495 is referred to as CIGR1 gene (its nucleotide sequence and amino acid sequence are shown in SEQ ID NOs: 1 and 2, respectively) and AU94860 is referred to as CIGR2 gene (its nucleotide sequence and amino acid sequence are shown in SEQ ID NOs: 3 and 4, respectively). The homologies between these two genes are 57% at the nucleotide level and 40% at the amino acid level. From the results of genomic Southern hybridization it is thought that a single copy of each of these genes is present (Fig. 3).

Fig. 4 depicts the presumed molecular phylogenetic tree of the CIGR1 and CIGR2 gene products and the GRAS family gene products with known structures. CIGR2 was thought to be rather closer to Arabidopsis AtSCL5 than to CIGR1. It was also suggested that gibberellin signal repressors form a single subfamily and that rice OsGAI (SLR) is closer to the corn repressor and the Arabidopsis repressor than CIGR1 gene and CIGR2 gene.

### [Example 2]

The products of the genes of the GRAS family are regarded as transcriptional regulators, but it is not known how they are involved in the regulation of gene expression. Arabidopsis SCARECROW does not have the typical nuclear localization signal. However, it is estimated to be a transcription factor because its N-terminal region is rich in serine, threonine, proline, and glutamine. GAI gene, which is one of the Arabidopsis gibberellin signal repressors, and GRS gene very similar to that, have nuclear localization signal-like sequences (Peng, J., Carol, P., Richards, D. E., King, K. E., Cowling, R. J., Murphy, G. P., and Harberd, N. P., Genes and Development (1997) 11, 3194-3205). Furthermore, RGA, which is another repressor, was shown to be localized in the nucleus by experiments using a GFP-fusion protein (Silverstone, A. L., Ciampaglio, C. N., and Sun, T.-P., "The Arabidopsis RGA gene encodes a transcriptional regulator repressing the gibberellin signal transduction pathway", The Plant Cell (1998) 10, 155-169). To analyze the intracellular localization of the CIGR1 gene and CIGR2 gene products, the chimeric plasmids in which their translation regions were connected in-frame to GFP, were introduced into onion epidermal cells using the particle gun method. Fusion protein behavior was monitored using GFP fluorescence. As a result, images demonstrating the localization of CIGR2-GFP fusion protein in the nucleus were observed (Fig. 5). Since such images were not observed when GFP only was introduced, it was concluded that the CIGR2 gene product was localized in the nucleus.

### [Example 3]

Both CIGR1 gene and CIGR2 gene were identified as elicitor responsive genes using DNA microarray analysis. The elicitor responsiveness of these two genes was analyzed using Northern blot hybridization method. Significant increases of mRNA amount were observed in both genes five minutes after treatment with chitin heptamer. Expression continued to increase for up to 90 minutes (Fig. 6a). It has been reported that the elicitor activity of chitin oligomers in rice depends on their size; that is, chitin heptamers and octamers have the strongest effects, and chitosan oligomers that are deacetylated derivatives have very low activities. Therefore, the present inventors studied the induction of expression of these two genes when the cells were treated by chitin oligomers , from monomer to heptamer, and chitosan oligomers, tetramer and heptamer. Both genes showed the strongest response to chitin heptamer. They had no significant response to chitosan oligomers (Fig. 6b).

### [Example 4]

Ashikari *et al*. (Ashikari, M., Wu, J., Yano, M., Sasaki, T., and Yoshimura, A., "Rice gibberellin-insensitive dwarf mutant gene Dwarf 1 encodes the alpha-subunit of GTP-binding protein", Proc. Natl. Acad. Sci. U. S. A. (1999) 96, 10284-10289) and Fujisawa *et al*. (Fujisawa, Y., Kato, T., Ishikawa, A., Kitano, H., Sasaki, T., Asahi, T., and Iwasaki, Y., "Suppression of the heterodimeric G protein causes abnormal morphology, including dwarfism, in rice", Proc. Natl. Acad. Sci. U. S. A. (1999) 96, 7575-7580) independently found that gene D1, which causes dwarf mutant d1 in rice, encodes the α-subunit of the trimeric G protein. In animal cells, trimeric G proteins have been known to be coupled with the seven-transmembrane receptors and play important roles in the transduction of extracellular information (Neer, E. J., "Heterotrimeric G proteins: organizers of transmembrane signals", Cell (1995) 50, 1011-1019).

On the other hand, analysis using their inhibitors and activators suggested that the G proteins were involved in the transduction of elicitor signals (Legendre, L., Heinsyein, P. F., and Low, P. S., "Evidence for participation of GTP-binding proteins in elicitation of the rapid oxidative burst in cultured soybean cells", J. Biol. Chem. (1992) 267, 20140-20147). However, some problems were pointed out; e.g., the specificity of these inhibitors was not necessarily clear (Ephritikhine, G., Pradier, J.-M., and Guern, J., "Complexity of GTP γS binding to tobacco plasma membranes", Plant Physiol. Biochem. (1993) 31, 573-584). Thus, no clear conclusion has been obtained concerning the involvement of the D1 gene product.

Tsukada *et al*. compared in detail the various elicitor responses of calluses derived from d1 strain seeds with those of the wild type, and demonstrated that there was no significant difference between them (Tsukada, K. , Ishizaka, M. , Fujisawa, Y. , Iwasaki, Y. , Yamaguchi, T., Minami, E., and Shibuya, N., "Rice receptor for chitin oligosaccharide elicitor does not couple to heterotrimeric G-protein: Elicitor responses of suspension cultured rice cells from Daikoku dwarf (d1) mutants lacking a functional G-protein α-subunit", Physiol. Plantrum (2002) 116, 373-382).

Preliminary experiments demonstrated that the time-courses of elicitor induction of CIGR1 gene and CIGR2 gene in the d1 strain were similar to those in the wild type.

On the other hand, Ueguchi-Tanaka *et al*. showed that D1 gene is involved in the transduction of gibberellin signals, using as an indicator the induction of α-amylase in aleurone in germinating seeds (Ueguchi-Tanaka, M., Fujisawa, Y., Kobayashi, M., Ashikari, M., Iwasaki, Y., Kitano, H., and Matsuoka, M., "Rice dwarf mutant d1, which is defective in the α subunit of the heterotrimeric G protein, affects gibberellin signal transduction", Proc. Natl. Acad. Sci. U. S. A. (2000) 97, 11638-11643). Prior to this study, Schumacher *et al*. (Schumacher, K., Schmitt, T., Rossberg, M., Schmitz, G., and Theres, K., "The Lateral suppressor (Ls) gene of tomato encodes a new member of the VHIID protein family", Proc. Natl. Acad. Sci. U. S. A. (1999) 96, 290-295) showed that the tomato lateral bud suppressor gene product belonged to the same gene family as SCARECROW. They pointed out the possibility of an interaction between this gene product and gibberellin in the discussion on its role in morphogenesis.

Therefore, the present inventors examined the gibberellin responsiveness of CIGR1 and CIGR2 genes. Suspension-cultured cells were treated with GA3, which is one of the active-type gibberellins, and changes in the expression of these two genes were analyzed over time. In both genes, there was no significant change in the amount of expression up to three hours after treatment. To maintain cell division activity, the medium used for culturing plant cells contained 2, 4-D, which is one of auxins that are not easily metabolized by plants. It was thought that 2,4-D might suppress the action of gibberellin. Therefore, the cells were pre-washed with 2, 4-D-free medium, and after two hours of pre-culture in the same medium, the cells were treated with GA3. Transient expression with a maximum at ten minutes after treatment was observed (Fig. 7).

In the prior art, studies on the transduction of gibberellin signals were carried out mostly by analyzing the induction of α-amylase in aleurone tissues in cereal seeds. The above-mentioned result is the first example that uses cultured cells. Responsiveness to gibberellin was then analyzed in more detail using the expressions of CIGR1 gene and CIGR2 gene in cultured cells as indicators.

Fig. 8 shows the effect of GA3 concentration on the induction of expression of these two genes. The induction of expression of both genes began to appear when GA3 treatment was at 10⁻⁶ M. Induction reached near saturation at 10⁻⁴ M. Vishnevetsky *et al*. reported that CHRC, a carotenoid-associated protein in chromoplasts of cucumber petals, required at least 10⁻⁷ M GA3 for induction by gibberellin, and that its expression level increased approximately linearly up to 10⁻⁴ M GA3 (Vishnevetsky, M. , Ovadis, M. , Itzhaki, H. , and Vainstein, A., "CHRC, encoding a chromoplast-specific carotenoid-associated protein, is an early gibberellic acid-responsive gene", J. Biol. Chem. (1997) 272, 24747-24750). Though there were differences in the materials and genes, they obtained similar results for effective GA3 concentrations.

### [Example 5]

Unlike auxins and cytokinins, gibberellins are defined, not by their physiological activities, but as compounds that have the *ent*-gibberellane skeleton. Therefore, there are large variations in their activities. In order to judge which of the physiological activity or the *ent*-gibberellane skeleton itself induces CIGR1 gene and CIGR2 gene expression, the induction of expression of these two genes by active type (GA1, GA3, and GA4) and inactive type (GA13 and GA17) (Crozier, A., Kuo, C. C., Durley, R. C., and Pharis, R. P., "The biological activities of 26 gibberellins in nine plant bioassays", Canadian J. Botany (1970) 48, 867-877.) gibberellins was analyzed. The results showed that only active type gibberellins induced the expression of both genes (Fig. 9). This result strongly suggests that the above-mentioned induction of CIGR1 and CIGR2 gene expression by gibberellin is through a signal transduction mediated by a gibberellin receptor.

### [Example 6]

Cultured plant cells are thought to be in an undifferentiated state in the presence of auxins. They grow under heterotrophic conditions as they are devoid of chloroplasts. Due to such factors, cultured cells are quite different from whole plants in their histological and physiological characteristics. Therefore, the possibility cannot be denied that the induction of CIGR1 and CIGR2 gene expression by gibberellin is a special phenomenon in cultured cells. Thus, the responses of these two genes to gibberellin were analyzed in green leaves of rice. The third and fourth leaves of rice, three weeks after seeding, were sprayed with GA3 (50 µM) and total RNA was extracted at certain intervals over time to analyze changes in the expression level of these genes. Both genes showed a very rapid transient expression with a maximum at 30 minutes after spraying (Fig. 10). This strongly suggested that the signaling via a gibberellin receptor, found in cultured cells, was not peculiar to cultured cells and also functioned in whole rice plants.

### [Example 7]

Kuo *et al*. (Kuo, A., Cappelluti, S., Cervantes-Cervantes, M., Rodriguez, M., and Bush, D. S., "Okadaic acid, a protein phosphatase inhibitor, blocks calcium changes, gene expression and cell death induced by gibberellin in wheat aleurone cells", The Plant Cell (1996) 8, 259-269.) found that the induction of α-amylase in the aleurone layer of wheat by gibberellin was specifically inhibited by okadaic acid, one of the protein phosphatase inhibitors. Okadaic acid is known to inhibit PP1 and PP2B among the protein phosphatases of animals.

On the other hand, protein kinase inhibitors, staurosporine and K252a, hardly inhibited induction, so it was presumed that phosphorylation of proteins, especially by protein phosphatases, was significantly involved in the signal transduction from gibberellin to the α-amylase gene.

In contrast, the data to date of the present inventors shows that the induction of expression of elicitor-responsive genes is strongly inhibited by pretreatment with K-252a (He, D.-Y., Yazaki, Y., Nishizawa, Y., Takai, R., Yamada, K., Sakano, K., Shibuya, N., and Minami, E., "Gene activation by cytoplasm acidification in suspension-cultured rice cells in response to the potent elicitor, N-acetylchitoheptaose", Mol. Plant-Microbe Int. (1998) 12, 1167-1174; Nishizawa, Y., Kawakami, A. , Hibi, T. , He, D.-Y., Shibuya, N., and Minami, E., "Regulation of the chitinase gene expression in suspension-cultured rice cells by N-acetylchitooligosaccharides: differences in the signal transduction pathways leading to the activation of elicitor-responsive genes", Plant Mol. Biol. (1999) 39, 907-914).

In addition, the effects of various inhibitors on the inductions by gibberellin and elicitors of CIGR1 gene and CIGR2 gene in the cultured rice cells were examined. Referring to the results of Kuo *et al*., cells pretreated with okadaic acid were tested. In these cells, while the induction of both genes by chitin heptamer was hardly inhibited, induction by GA3 was inhibited almost completely (Fig. 11B). From these observations, protein phosphatase was presumed to be involved in signal transduction from gibberellin to CIGR1 gene and CIGR2 gene in cultured rice cells. Following these experiments, the effects of protein kinase inhibitors were studied. Lavendustin A, which is known as an inhibitor of the receptor-type protein tyrosine kinase, exhibited a pattern of inhibition similar to that of okadaic acid. Lavendustin A hardly inhibited induction by chitin heptamer, but inhibited induction by GA3 almost completely (Fig. 11C). K-252-A, which is supposed to inhibit both protein serine/threonine kinase and protein tyrosine kinase, inhibited the inductions of both genes by chitin heptamer and GA3 almost completely (Fig. 11D).

The induction of α-amylase by gibberellin in the aleurone layer of wheat is reported to be inhibited by okadaic acid but not by K-252-A (Kuo, A., Cappelluti, S., Cervantes-Cervantes, M., Rodriguez, M., and Bush, D. S., "Okadaic acid, a protein phosphatase inhibitor, blocks calcium change, gene expression and cell death induced by gibberellin in wheat aleurone cells", The Plant Cell (1996) 8, 259-269).

The results shown in Fig. 11 suggest that, in addition to protein phosphatase, which is inhibited by okadaic acid, protein kinase, which is inhibited by K-252a and lavendustin A, is involved in gibberellin induction of CIGR1 gene and CIGR2 gene in cultured cells. There were similar and different aspects between the signal transduction in the cultured cells and that in the aleurone layer. Signal transduction from gibberellin is thought to be qualitatively different from that from chitin heptamer.

Richards *et al*. (Richards, D. E., Peng, J., and Harberd, N. P., BioEssays (2000) 22, 573-577) have proposed a hypothesis that the GRAS family corresponds to STATs, which are transcriptional factors found widely in metazoan organisms. In the C-terminal regions of GRAS, structures similar to the SH2 region are found; the SH2 region is a structure common in the STAT family, transcription factors in animals. Near the C-terminal of the SH2 region, a tyrosine residue that is highly conserved in animal STATs and susceptible to phosphorylation, and an arginine residue that is supposed to interact electrostatically with the phosphorylated tyrosine, are conserved. The homology in the N-terminal regions of amino acid sequences is lower than that in the C-terminal regions. This is generally found in the GRAS family. The homology of CIGR1 gene and CIGR2 gene at the nucleotide level was 57%. From the result of genomic Southern hybridization, it was estimated that one copy of each of these two genes was present (Fig. 3). Five µg of rice genomic DNA were completely digested by restriction enzyme BamHI (B), EcoRI (E), or HindIII (H). The products were separated on agarose gel by electrophoresis and transferred onto nitrocellulose membrane. Hybridization with the ³²P-labelled CIGR1 and CIGR2 gave patterns consistent with cDNA maps.

From these results, each of the full length strand cDNAs was thought to hybridize specifically with its corresponding gene product. Both genes do not comprise the DELLA sequence, a common structure in the GAI/RGA subfamily which is a negative regulator of gibberellin signals.

### Industrial Applicability

The present invention provides plant elicitor- and gibberellin-responsive genes. In plants, elicitors are known to induce a variety of defense-related enzyme genes, bringing about a defense reaction. Thus, it is highly expected that CIGR1 and GIGR2 genes, which can be induced by elicitors, will be valuable in disease-resistant recombinant crops.

## Claims

1. A DNA encoding a plant protein, wherein the DNA is any one of (a) to (d):
(a) a DNA comprising the nucleotide sequence of SEQ ID NO: 1 or SEQ ID NO: 3;
(b) a DNA that hybridizes under stringent conditions with the DNA comprising the nucleotide sequence of SEQ ID NO: 1 or SEQ ID NO: 3;
(c) a DNA comprising the amino acid sequence of SEQ ID NO: 2 or SEQ ID NO: 4;
(d) a DNA comprising the amino acid sequence of SEQ ID NO: 2 or SEQ ID NO: 4, in which one or more amino acids have been substituted, deleted, added, and/or inserted.

2. The DNA of claim 1, wherein the plant is rice.

3. A DNA encoding a protein that comprises a characteristic dominant negative for the protein coded by the DNA of claim 1 or 2.

4. A protein coded by a DNA of any one of claims 1 to 3.

5. A nucleic acid of any one of (a) to (d):
(a) an antisense nucleic acid complementary to the transcription product of the DNA of claim 1 or 2;
(b) a nucleic acid comprising the ribozyme activity of specifically cleaving the transcription product of the DNA of claim 1 or 2;
(c) a nucleic acid comprising the effect of inhibiting the expression of the DNA of claim 1 or 2 by co-suppression;
(d) a nucleic acid comprising the effect of inhibiting the expression of the DNA of claim 1 or 2 by an RNAi effect.

6. A vector comprising the nucleic acid of claim 5 or a DNA of any one of claims 1 to 3.

7. A transformed plant cell that maintains a DNA of any one of claims 1 to 3, the nucleic acid of claim 5, or the vector of claim 6.

8. A transformed plant comprising the transformed plant cell of claim 7.

9. The transformed plant of claim 8, wherein the plant is rice-derived.

10. A transformed plant that is an offspring or clone of the transformed plant of claim 8 or 9.

11. A reproductive material of a transformed plant of any one of claims 8 to 10.

12. A method for producing a transformed plant of any one of claims 8 to 10, which comprises the step of inserting a DNA of any one of claims 1 to 3, the nucleic acid of claim 5, or the vector of claim 6 into a plant cell to reproduce a plant from the plant cell.

13. The method of claim 12, wherein the plant is rice.
